# EUROPEAN PATENT APPLICATION

(11) **EP 4 349 336 A1**
(43) Date of publication of application: **10.04.2024**
(21) Application number: 22306483.3
(22) Date of filing: 04.10.2022
(51) Int. Cl.: A61K 31/4709, A61K 31/519, A61K 31/4184, A61K 31/4523, A61P 35/00

(54) **COMBINATION OF SUBSTITUTED 2,4 DIAMINO-QUINOLINE COMPOUNDS AND MEK INHIBITORS FOR USE IN THE TREATMENT OF LIVER CANCERS**

(71) Applicant: Genoscience Pharma, 13006 Marseille (FR)
(72) Inventor: HALFON, Philippe, 13008 Marseille (FR); BESTION, Eloïne, 13009 Marseille (FR); MEZOUAR, Soraya, 13014 Marseille (FR); COURCAMBECK, Jérôme, 13009 Marseille (FR); MENUT, Agnès, 13012 Marseille (FR)
(74) Representative: Santarelli

(57) **Abstract**

The invention relates to a combination of substituted 2,4 diamino-quinoline compounds and MEK inhibitors, pharmaceutical compositions containing it and its use as a medicament. The invention further relates to a combination of substituted 2,4 diamino-quinoline compounds and MEK inhibitors, for use in the prevention and/or decreasing and/or treatment of liver cancers. The substituted 2,4 diamino-quinoline compounds and MEK inhibitors constituting said combination can be used simultaneously, separately or sequentially.

## Description

### Field of the invention

The invention relates to a combination of substituted 2,4 diamino-quinoline compounds and MEK inhibitors, pharmaceutical compositions containing it and its use as a medicament. The invention further relates to a combination of substituted 2,4 diamino-quinoline compounds and MEK inhibitors, for use in the prevention and/or decreasing and/or treatment of liver cancers. The substituted 2,4 diamino-quinoline compounds and mitogen-activated protein kinase kinase inhibitors (MEK inhibitors) constituting said combination can be used simultaneously, separately or sequentially.

### Background of the invention

KRAS mutations represent a driver event of pancreatic ductal adenocarcinoma (PDAC). Still today targeting non wild-type KRAS in cancer therapy has proven to be challenging. However, targeting oncogene-driven signaling pathways represents a clinically validated approach in several cancers (e.g. chronic myelogenous leukemia, melanoma). It is described that a subpopulation of dormant tumor cells surviving KRAS ablation and responsible for tumor relapse depend on the mitochondrial function, lysosome activity and on the activation of the autophagy for survival. Moreover, inhibition of MEK→ERK signaling pathway leads to a high dependence of PDAC cells on autophagy for persistence. The mitogen-activated protein kinase kinase inhibitors (MEK inhibitors) forces and drives cancer cells to be highly depend on the cellular autophagy for survival. Thereby, the MEK inhibitors may induce an autophagy dependence for survival of cancer cells, which is an easy way for cancer cells to acquire drug resistance to MEK inhibitors in the oncology clinical setting.

Several studies have highlighted the links between the use of MEK inhibitors and the consequences on the autophagy pathway. It was found that PDAC are resistant to the Trametinib (a MEK inhibitor) monotherapy or chloroquine/hydroxychloroquine (autophagy inhibitor) monotherapy, but are highly sensitive to the dual therapy Trametinib + chloroquine at the preclinical level and to the dual therapy Trametinib + hydroxychloroquine at the clinical level. Inhibition of KRAS → RAF → MEK → ERK signaling pathway induces the cellular autophagy which is responsible of cellular recycling that protects PDAC from the cytotoxic effects of the KRAS pathway inhibition (e.g. using a MEK inhibitor). The inhibition of mitogen-activated protein kinase kinases 1/2 (MEK1/2 kinases) leads to the activation of the LKB1→AMPK→ULK1 signaling pathway, which is a key regulator of the cellular autophagy. The induced cellular autophagy in response to the KRAS inhibition pathway was equally observed by the inhibition of the KRAS downstream ERK effector. By the same way, it was found that the inhibition of the cellular autophagy using an autophagy inhibitor like chloroquine or by the use of genetic or pharmacologic autophagy inhibition enhanced the ability of ERK inhibitors to mediate antitumor response in KRAS-driven pancreatic cell. Altogether these data show that inhibition of ERK signaling pathway drives cancer cells to become acutely dependent on cellular autophagy process in order to acquire ERK drug resistance.

These studies are consistent with previous observations that autophagy serves as an adaptive and protective response to the inhibition of KRAS → RAF → MEK → ERK signaling in cancer. Autophagy has also implicated in resistance to numerous other standard anticancer chemotherapies. For example, it was found that autophagy induction is a cause of ovarian cancer drug resistance to the highly cytotoxic agent paclitaxel. By the same way of the autophagy induction, ovarian and esophageal cancers can acquire the capacity to evade to the cisplatin therapy. Cisplatin lung cancer drug resistance can equally be acquired by hypoxia induced autophagy. In some other cancers, autophagy induction associated to the reticulum endoplasmic stress response results to cyclin dependent kinase inhibitors drug resistance in primary patient chronic lymphocytic leukemia derived cells, and results also to HDAC inhibitor (eg. Tabastatin A) drug resistance in glioblastoma cell lines. Autophagy is particularly active during metabolic stress that occurs in tumor microenvironment and is a critical survival pathway for cancer cells under other stress conditions making of the autophagy a cellular partner for tumor growth.

Hepatocellular carcinoma (HCC) is an aggressive malignancy, and is the most common primary liver cancer, characterized by high biological aggressiveness and with limited effective treatment options. An important signaling pathway in hepatocarcinogenesis is the MEK cascade involved in various cellular responses, including adaptation and survival. A key role in this cascade is played by MEK, of which MEK 1/2 represent the prototypes and an interesting therapeutic target for new oncological drugs. MEK 1/2 inhibitors represent novel targeted therapies in the management of advanced hepatocellular carcinoma (HCC) patients. However, the inhibition of the KRAS → RAF → MEK → ERK signaling pathway induces autophagy in some cancer models which is implicated in the tumor survival and drug resistance selection. Therefore, the inclusion of a MEK inhibitor in HCC anticancer therapy may result in rapidly losing a robust anticancer response in clinical setting by the acquisition of MEK inhibitor drug resistance. In the hepato-oncology field, the intrahepatic cholangiocarcinoma (iCCA) is the most common biliary malignancy. Surgical resection or liver transplantation are potentially curative treatment options for patients with early stage disease. However, most patients present with advanced-stage disease and therapeutic options are limited. The acquired mutations of KRAS occur in approximately 20% of iCCAs. Activated mutations of KRAS lead to the hyperactivation of the KRAS → RAF → MEK → ERK pathway with consequent augmentation of cell proliferation and survival. MEK inhibition has demonstrated efficacy in both wild-type and mutated KRAS (eg. KRAS V12D).

There is thus a need to treat liver cancers, including hepatocellular carcinoma, hepatoblastoma, intrahepatic cholangiocarcinoma or extrahepatic cholangiocarcinoma while maintaining a robust and durable response to anticancer therapy, in particular by avoiding acquisition of drug resistance during MEK inhibitor therapy in clinical setting.

### Summary of the invention

It has now been found that the combination of a substituted 2,4 diamino-quinoline compound and MEK inhibitors made it possible to obtain not only an overall additive effect, but also significant synergy with respect to inhibiting the growth of hepatic cancer cells, with a potential significant impact *in vivo* and in clinical setting. These results open a new way to overcome potential MEK inhibitor drug resistance issues in liver anticancer therapy.

The invention relates to a combination comprising
◆ a compound of formula (I) 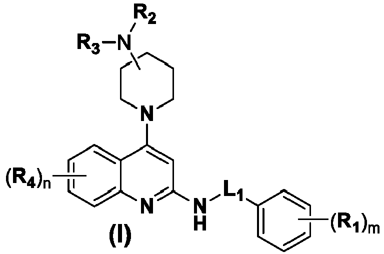 and pharmaceutically acceptable salts, hydrates, solvate, prodrugs, polymorphs, tautomers, isotopic variants, isomers, stereoisomers or mixtures of stereoisomers thereof, and
◆ a Mitogen-Activated Protein Kinase Kinase inhibitor (MEK inhibitor) and pharmaceutically acceptable salts, hydrates or solvates thereof.

The invention also relates to pharmaceutical compositions containing this combination, where each component of the combination can be contained in a separate pharmaceutical composition.

Another object of the invention is a combination of a compound of formula (I) and a MEK inhibitor for use in the treatment and/or prevention and/or decrease of liver cancers related diseases, in particular hepatocarcinoma, hepatoblastoma or chlangiocarcinoma.

The compound of formula (I) and the MEK inhibitor, or the pharmaceutical composition(s) containing them, can be administered simultaneously, separately or sequentially.

The invention further relates to a kit comprising the combination of a compound of formula (I) and a MEK inhibitor, in which both or either of the components are/is in the form of a pharmaceutical composition which may be administered simultaneously, separately or sequentially.

### Detailed description of the invention

The invention relates to a combination comprising
◆ a compound of formula (I) 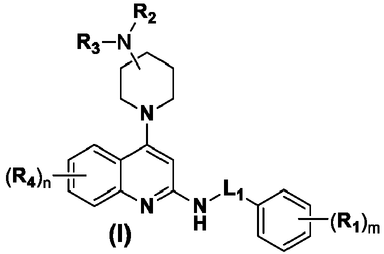 wherein
   - **L₁** is chosen from a single bond; an optionally substituted (CH₂)p group; an optionally substituted alkylene; a carbonyl.
   - **R₁** , either alone or simultaneously or independently when m > 1 is selected from a hydrogen atom; a halogen atom ; an optionally substituted alkyl; a haloalkyl; a fluoroalkyl; an optionally substituted alkenyl; an optionally substituted alkynyl; an optionally substituted cycloalkyl; an optionally substituted cycloalkenyl; an optionally substituted cycloalkynyl; an optionally substituted alkoxy; -O-**R₇**; -O-(CO)-**R₇**; -O-(CO)-N**R₅R₆**; -N**R₅**-(CO)-**R₇**; -O-(CO)-O**R₇**; -N**R₅**-(CO)-O-**R₇**; an azido; a hydroxyl; a cyano; a nitro; -N**R₅R₆**;
   - **R₂** and **R₃** , simultaneously or independently, are selected from a hydrogen atom; an optionally substituted alkyl; an optionally substituted alkenyl; an optionally substituted alkynyl; an optionally substituted cycloalkyl; an optionally substituted cycloalkenyl; an optionally substituted cycloalkynyl; an optionally substituted heterocyclyl group; an optionally substituted aryl; an optionally substituted benzyl; an optionally substituted heteroaryl;
      or **R₂** and **R₃** can be linked together with the nitrogen atom to which they are covalently linked to form an optionally substituted heterocyclyl group;
   - **R₄** , either alone or simultaneously or independently when n > 1, is selected from a hydrogen atom; a halogen atom; an optionally substituted alkyl; a haloalkyl; a fluoroalkyl; an optionally substituted alkenyl; an optionally substituted alkynyl; an optionally substituted cycloalkyl; an optionally substituted cycloalkenyl; an optionally substituted cycloalkynyl; an optionally substituted alkoxy; -O-**R₇**; -O-(CO)-**R₇**; -O-(CO)-N**R₅R₆**; -N**R₅**-(CO)-**R₇**; -O-(CO)-O**R₇**; -N**R₅**-(CO)-O-**R₇**; an azido; a hydroxyl; a cyano; a nitro; -N**R₅R₆**.
   - **R₅** and **R₆** , simultaneously or independently, are slected from a hydrogen atom; an optionally substituted alkyl; an optionally substituted alkenyl; an optionally substituted alkynyl; an optionally substituted cycloalkyl; an optionally substituted cycloalkenyl; an optionally substituted cycloalkynyl; an optionally substituted heterocyclyl group; an optionally substituted aryl; an optionally substituted heteroaryl ;
      or **R₅** and **R₆** can be linked together with the nitrogen atom to which they are covalently linked to form an optionally substituted heterocyclyl group ;
   - **R₇** can be selected from an optionally substituted alkyl; a haloalkyl; a fluoroalkyl; an optionally substituted alkenyl; an optionally substituted alkynyl; an optionally substituted cycloalkyl; an optionally substituted cycloalkenyl; an optionally substituted cycloalkynyl; an optionally substituted heterocyclyl group; an optionally substituted aryl; an optionally substituted heteroaryl;
   - n is an integer which can have any one of the values 0, 1, 2, 3 or 4;
   - m is an integer which can have any one of the values 0, 1, 2, 3, 4 or 5;
   - p is an integer which can have any one of the values 0 or 1;
      and its pharmaceutically acceptable salts, hydrates, solvates, prodrugs, polymorphs, tautomers, isotopic variants, isomers, stereoisomers or mixtures of stereoisomers, and
◆ a Mitogen-Activated Protein Kinase Kinase inhibitor (MEK inhibitor) and pharmaceutically acceptable salts, hydrates or solvates thereof.
   ◆ The terms "bond" or "single bond" refer to a chemical bond between two atoms, or two moieties when the atoms joined by the bond are considered to be part of larger substructure.
   ◆ The expression "simultaneously or independently" is used herein to indicate that a variable is applied in any one instance without regard to the presence or absence of a variable having that same or a different definition within the same compound. Thus, for instance, in a compound in which a substituent Xi appears twice and is defined as "simultaneously or independently, group G1 or group G2", both Xi can be G1 (simultaneously), both Xi can be G2 (simultaneously), or one Xi can be G1 and the other G2 (independently).
   ◆ The expressions "halogen", "halogen atom", "halogens" or "halogen atoms" everywhere it appears mean an or more atom(s) that can be chosen from fluorine (F), chlorine (CI), bromine (Br), iodine (I), preferably a fluorine, a chlorine or a bromine and more preferably a fluorine or a chlorine.
   ◆ The term "alkyl", alone or in combination with other groups, refers to a branched or linear chain monovalent saturated aliphatic hydrocarbon radical of 1 to 20 carbon atoms (C₁-C₂₀ carbon atoms), preferably 1 to 16 carbon atoms (C₁-C₁₆ carbon atoms), more preferably lower alkyl of 1 to 10 carbon atoms (C₁-C₁₀ carbon atoms). Alkyl groups can be optionally substituted as defined herein.
   ◆ The term "alkylene " refers to an alkyl group as described above, where alkyl is diradical also defined as alkanediyl. Typically, an alkylene group has two points of attachment to the rest of the molecule (e.g. -L₁- in the molecule represented by formula (I) of the present invention). The two points of attachment of the alkylene group can be located on one specific carbon atom or on two different carbons atoms thereof.
   ◆ The term "lower alkyl", alone or in combination, denotes; a linear or branched-chain alkyl group with 1 to 10 carbon atoms ("C₁-C₁₀-alkyl"), preferably a linear or branched-chain alkyl group with 1 to 5 carbon atoms ("C₁-C₅-alkyl"), and particularly preferred a linear or branched-chain alkyl group with 1 to 3 carbon atoms ("C₁-C₃-alkyl"). Lower alkyl groups can be optionally substituted as defined herein. Non-limiting examples of linear and branched lower alkyl groups are methyl, ethyl, *n*-propyl, isopropyl, *n*-butyl, isobutyl, *sec*-butyl, *tert*-butyl, the isomeric pentyls, the isomeric hexyls, the isomeric heptyls, the isomeric octyls, the isomeric nonyls, the isomeric decanyls, preferably methyl and ethyl and propyl and isopropyl and *tert-*butyl and isobutyl and *sec*-butyl and the isomeric pentyls and most preferred methyl and ethyl and n-propyl and isopropyl, and n-butyl and *tert*-butyl.
   ◆ The term "alkenyl" denotes a linear or branched chain hydrocarbon residue comprising an olefinic bond 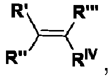 wherein **R'**, **R"**, **R‴** and **R^{IV}** refer to the remaining portions of the alkenyl group, which may be the same or different. The **R', R", R‴** and **R^{IV}** portion of the alkenyl moiety may be branched, linear chain, or cyclic. Alkenyl groups can have 2 to 10 carbon atoms ("C₂-C₁₀-alkenyl"), preferably 2 to 5 carbon atoms ("C₂-C₅-alkenyl"), particularly preferred 2 to 4 carbon atoms ("C₂-C₄-alkenyl"). The alkenyl moiety may be branched, linear chain, or cyclic (in which case, it would also be known as a "cycloalkenyl" group). The alkenyl group could also be a "lower alkenyl" having 2 to 6 carbon atoms including all isomeric forms (*cis, trans*, *Z*, *E*). Alkenyl groups can be optionally substituted as defined herein. Non-limiting examples of lower alkenyl groups are ethenyl, 1-propenyl, 2-propenyl, 1-butenyl, 2-butenyl, isobutenyl, 1-pentenyl, 2-pentenyl, 3-pentenyl, 3-penten-2-yl, 3-penten-4-yl isopentenyl, 1-hexenyl, 2-hexenyl, 3-hexenyl, 4-hexenyl, isohexenyl. Preferred examples are ethenyl, 1-propenyl, 2-propenyl, 2-butenyl, 2-buten-2-yl and isopentenyl.
   ◆ The term "alkynyl" denotes a linear or branched chain hydrocarbon residue comprising an alkyne bond where two carbon atoms form a triple bond R'-C≡C-R", wherein R' and R" refer to the remaining portions of the alkynyl group, which may be the same or different. The R' and R" portion of the alkynyl moiety may be branched, linear chain, or cyclic. Alkynyl groups can have 2 to 10 carbon atoms ("C₂-C₁₀-alkynyl"), preferably 2 to 5 carbon atoms ("C₂-C₅-alkynyl"), particularly preferred 2 to 4 carbon atoms ("C₂-C₄-alkynyl"). Alkynyl groups can be optionally substituted as defined herein. Non limiting examples of alkynyl groups are, ethynyl, 1-propynyl, 2-propynyl, 1-butynyl, 3-butynyl, 4-butynyl, but-2-yn-1-yl, 1-pentynyl, pent-2-yn-1-yl, pent-3-yn-1-yl, pent-4-yn-1-yl, pent-2-yn-3-yl. Preferred examples are propyn-1-yl, propyn-3-yl, butyn-1-yl, butyn-3yl, butyn-4-yl, but-2-yn-1-yl. The alkynyl group could also be a "lower alkynyl" having 2 to 6 carbon atoms ("C₂-C₆-alkynyl").
   ◆ The term "cycloalkyl" denotes a monocyclic or polycyclic radical that contains only carbon and hydrogen, being saturated rings and containing from 3 to 12 carbon atoms "C₃-C₁₂-cycloalkyl", such as cyclopropyl, cyclobutyl, cyclopentyl, cyclohexyl, cycloheptyl, cyclooctyl, cyclononyl or cyclodecane. Cycloalkyl groups include groups having from 3 to 12 ring atoms "C₃-C₁₂-cycloalkyl", preferably from 3 to 8 ring atoms "C₃-C₈-cycloalkyl" and more preferably from 3 to 7 ring atoms "C₃-C₇-cycloalkyl" and still more preferably from 3 to 6 ring atoms "C₃-C₆-cycloalkyl". Depending on the structure, a cycloalkyl group can contain an adjacent and substituting cycloalkenyl and/or alkenyl group. Cycloalkyl groups can be optionally substituted as defined herein.
   ◆ The term "cycloalkenyl" means a non-aromatic mono or multicyclic ring system comprising about 3 to about 12 carbon atoms, preferably about 5 to about 10 carbon atoms, and more preferably about 5 to about 7 carbon atoms, and stil more preferably about 5 to about 6 carbon atoms which contains at least one carbon-carbon double bond C=C. The cycloalkenyl can be optionally substituted with one or more "ring system substituents" which may be the same or different, and are as defined above. Non-limiting examples of monocyclic cycloalkenyls include cyclopentenyl, cyclohexenyl, cyclohepta-1,3-dienyl, and the like. Non-limiting example of a suitable multicyclic cycloalkenyl is for example norbornylenyl, and the like. Cycloalkenyl groups can be optionally substituted as defined herein.
   ◆ The term "cycloalkynyl" means a non-aromatic mono or multicyclic ring system comprising about 8 to about 12 carbon atoms, preferably about 8 to about 10 carbon atoms which contains at least one carbon-carbon triple bond (C≡C). The cycloalkynyl can be optionally substituted with one or more "ring system substituents" which may be the same or different, and are as defined above. Non-limiting examples of monocyclic cycloalkynyls include cyclooctynyl, cyclononynyl, cyclodecynyl and the like. Cycloalkynyl groups can be optionally substituted as defined herein.
   ◆ The terms "haloalkyl," "haloalkenyl," "haloalkynyl" and "haloalkoxy" include alkyl, alkenyl, alkynyl and alkoxy structures as defined herein in which at least one hydrogen is replaced with a halogen atom as defined herein. In certain embodiments in which two or more hydrogen atoms are replaced with halogen atoms, the halogen atoms are all the same as one another. In other embodiments in which two or more hydrogen atoms are replaced with halogen atoms, the halogen atoms are all the same or not all the same as one another.
   ◆ The term "fluoroalkyl," as used herein, refers to linear or branched-chain alkyl group as defined herein in which at least one hydrogen is replaced with a fluorine atom. Examples of fluoroalkyl groups include, but are not limited to -CH₂F, -CHF₂, -CF₃, -CH₂CF₃, - CF₂CF₃, -CF₂CH₃, -CH₂CH₂CF₃, -CH(CF₃)₂, -CF₂CH(CH₃)₂ and the like. The "fluoroalkyl" can be optionally substituted as defined herein.
   ◆ An "alkoxy" group refers to a -O(C₁-C₁₀ alkyl) group, -O(cycloalkyl) group, - O(heterocyclyl) group, wherein "C₁-C₁₀ alkyl", "C₁-C₁₀ cycloalkyl" and "heterocyclyl" is as defined above. The term "lower alkoxy" refers to the group R'-O-, wherein R' is lower alkyl and the term "lower alkyl" has the previously given significance. Alkoxy groups can be optionally substituted as defined herein. Non limiting examples of lower alkoxy groups are methoxy, ethoxy, n-propoxy, isopropoxy, n-butoxy, isobutoxy, *sec*-butoxy and *tert*-butoxy, *n*-pentoxy, *n-*hexyloxy, preferably methoxy and ethoxy and isopropoxy and *tert*-butoxy most preferred methoxy and ethoxy. Non limiting examples of "alkoxy" having -O(cycloalkyl) group are cyclopropyloxy, cyclobutyloxy, cylopentyloxy, cyclohexyloxy.
   ◆ The terms "heterocyclic group", "non-aromatic heterocycle", "heterocycloalkyl", "heterocyclyl" or "heteroalicyclic" refer to a fully saturated or unsaturated but not fully unsaturated, being a 3 to 9 membered monocyclic groups, preferably from 3 to 7 membered monocyclic groups and more preferably from 3 to 6 membered monocyclic groups or fused heterocyclic ring systems comprising from 5 to 16 atoms, preferably from 5 to 14 atoms, and more preferably from 5 to 10 atoms and still more preferably from 5 to 9 which have at least one heteroatom, said heteroatom being, when more than one, simultaneously or independently, chosen from oxygen atom, nitrogen atom or sulfur atom. Each ring of the heterocyclic group can have at least one heteroatom, said heteroatom being, when more than one, simultaneously or independently, chosen from nitrogen atoms, oxygen atoms and/or sulphur atoms. "Heterocycloalkyl" can be optionally substituted as defined herein. Covalent binding to a "heterocycloalkyl" can be at a heteroatom or *via* a carbon atom. In certain embodiments, non-aromatic heterocycles contain one or more carbonyl or thiocarbonyl groups such as, for example, oxo- and thio-containing groups. Heterocyclic groups can be optionally substituted as defined herein. Examples of heterocycloalkyls include, but are not limited to, lactams, lactones, cyclic imides, cyclic thioimides, cyclic carbamates, cyclic urea, tetrahydrothiopyran, 4H-pyran, tetrahydropyran, piperidine, 1,3-dioxin, 1,3-dioxane, 1,4-dioxin, 1,4-dioxane, piperazine, 1,3-oxathiane, 1,4-oxathiin, 1,4-oxathiane, tetrahydro-1,4-thiazine, 2H-1,2-oxazine, maleimide, succinimide, barbituric acid, thiobarbituric acid, dioxopiperazine, hydantoin, dihydrouracil, morpholine, trioxane, hexahydro-1,3,5-triazine, tetrahydrothiophene, tetrahydrofuran, pyrroline, pyrrolidine, pyrrolidone, pyrrolidione, pyrazoline, pyrazolidine, imidazoline, imidazolidine, 1,3-dioxole, 1,3-dioxolane, 1,3-dithiole, 1,3-dithiolane, isoxazoline, isoxazolidine, oxazoline, oxazolidine, oxazolidinone, thiazoline, thiazolidine, 1,3-oxathiolane and N-oxides thereof for amino heterocycle.
   ◆ The term "aryl" refers to an aromatic ring wherein each of the atoms forming the ring is a carbon atom. Aryl rings can contain five, six, seven, eight, nine, or more than nine carbon atoms, preferably from 5 to 16 carbon atoms, more preferably from 5 to 12 carbon atoms, and still more preferably from 5 to 10 carbon atoms and mean any stable monocyclic, bicyclic and tricyclic ring systems wherein at least one ring is aromatic. Aryl groups can be optionally substituted as defined herein. Examples of aryl groups include, but are not limited to phenyl, naphthalenyl, phenanthrenyl, anthracenyl, fluorenyl, biphenyl, and indenyl. Depending on the structure, an aryl group can be a monoradical or a diradical in which case it would be known as an arylene group. Examples of arylene groups include, but are not limited to, benzene-1,3-diyl, benzene-1,4-diyl, naphthalene-2,7-diyl, naphthalene-2,6-diyl, naphthalene-1,4-diyl naphthalene-1,5-diyl, acenaphthene-diyl, phenanthren-3,8-diyl, fluoranthene-diyl, 3-methylbenzene-1,4-diyl and the like.
   ◆ The term "heteroaryl" in general refers to an aromatic 5- or 11-membered ring which comprises at least one heteroatom and can in addition comprise one, two, three or four atoms chosen from nitrogen, oxygen and/or sulphur, such as pyridyl, pyrazinyl, pyrimidinyl, pyridazinyl, 2-oxo-l,2-dihydropyridinyl, oxadiazolyl, isoxazolyl, thiadiazolyl, triazolyl, tetrazolyl pyrazolyl, imidazolyl, thiophenyl, furanyl, oxazolyl, isothiazolyl, and thiazolyl. The term "heteroaryl" further refers to bicyclic aromatic or partly unsaturated groups comprising two 5- or 6-membered rings, in which one or both rings can contain one, two, three or four atoms chosen from nitrogen, oxygen or sulphur, such as quinolinyl, isoquinolinyl, cinnolinyl, pyrazolyl, imidazolyl, thiazolyl, thiophenyl, furanyl, oxazolyl, isothiazolyl, pyrazolo[1,5-*a*]pyridyl, imidazo[1,2-*a*] pyridyl, quinoxalinyl, quinazolyl, benzothiazolyl, benzotriazolyl, 1H-benzo[d]imidazole, benzo[*d*]isoxazolyl, benzo[*d*]isothiazolyl, benzo[c]isoxazolyl, benzo[c]isothiazolyl, indolyl, isoindolinyl, 6,7-dihydro-5H-pyrrolo[3,4-b]pyridinyl, 2,3-dihydro-1H-pyrrolo[3,4-c]pyridinyl, 6,7-dihydro-5H-pyrrolo[3,4-d]pyrimidinyl, purinyl, indazolyl, indolizinyl, imidazo[1,2-*a*]pyridinyl, imidazo[1,5-*a*]pyridinyl, imidazo[1,5-*a*]pyrazinyl, imidazo[1,2-*a*]pyrazinyl, 1H-imidazo[4,5-*b*]pyrazinyl, pyrazolo[1,5-*a*]pyridinyl, pyrrolo[1,2-*a*]pyrimidinyl, pyrrolo[1,2-*a*]pyrazinyl, pyrrolo[1,2-*c*]pyrimidinyl, oxazolo[4,5-*b*]pyridinyl, oxazolo[4,5-*c*]pyridinyl, oxazolo[5,4-*c*]pyridinyl, oxazolo[5,4-*b*]pyridinyl, thiazolo[4,5-*b*]pyridinyl, thiazolo[4,5-*c*]pyridinyl, thiazolo[5,4-*c*]pyridinyl, thiazolo[5,4-*b*]pyridinyl, oxazolo[5,4-*d*]pyrimidinyl, oxazolo[4,5-*d*]pyrimidinyl, thiazolo[5,4-*d*]pyrimidinyl, thiazolo[4,5-*d*]pyrimidinyl, oxazolo[4,5-*b*]pyrazinyl, thiazolo[4,5-*b*]pyrazinyl, isoxazolo[4,5-*b*]pyrazinyl, isothiazolo[4,5-*b*]pyrazinyl, isoxazolo[4,5-*d*]pyrimidinyl, isothiazolo[4,5-*d*]pyrimidinyl, isoxazolo[5,4-*d*]pyrimidinyl, isothiazolo[5,4-*d*]pyrimidinyl, isoxazolo[5,4-*b*]pyridinyl, isothiazolo[5,4-*c*]pyrimidinyl, isoxazolo[5,4-*c*]pyridinyl, isothiazolo[4,5-*c*]pyridinyl, isoxazolo[4,5*-*c]pyridinyl, isoxazolo[4,5-*b*]pyridinyl, isoxazolo[4,3-*d*]pyrimidinyl, isthiazolo[4,3-*d*]pyrimidinyl, isoxazolo[3,4-*d*]pyrimidinyl, isothiazolo[3,4-*d*]pyrimidinyl, pyrido[2,3-*d*]pyrimidinyl, pyrido[3,4-*d*]pyrimidinyl, pyrido[4,3-*d*]pyrimidinyl, pyrido[3,2-*d*]pyrimidinyl, pyrido[2,3-*b*]pyrazinyl, pyrido[3,4-*b*]pyrazinyl, [1,2,3]triazolo[4,5-*b*]pyridinyl, [1,2,3]triazolo[4,5-*c*]pyridinyl, 3H-[1,2,3]triazolo[4,5-*d*]pyrimidinyl. Preferred heteroaryl groups are pyridyl, thiozolyl, isothiazolyl, oxazolyl, isoxazolyl, quinozolinyl, and pyrazinyl.
   ◆ The terms "heteroaryl" or, alternatively, "heteroaromatic" refers to an aryl group that includes one or more ring and one or more heteroatoms independently, selected from nitrogen (N), oxygen (O) and sulfur (S). An N-containing "heteroaryl" or "heteroaromatic" moiety refers to an aromatic group in which at least one of the skeletal atoms of the ring is a nitrogen atom (N). Heteroaryl groups can be optionally substituted as defined herein.
   ◆ The terms "heteroaryl" or, alternatively, "heteroaromatic" also refers equally to fused heteroaryl systems wherein two or more rings share one or more bonds comprising from 7 to 16 ring atoms, preferably from 8 to 13 ring atoms, more preferably from 8 to 10 ring atoms comprising 1, 2, 3, 4 or 5 heteroatoms simultaneously or independently selected from nitrogen (N), oxygen (O) and sulfur (S) and comprising at least one carbon atom (C), provided that the fused rings do not include adjacent oxygen (O) and/or sulfur (S) atoms. Said "heteroaryl" can be optionally substituted as defined herein. Covalent binding to a heteroaryl can be at a heteroatom or *via* a carbon atom. N-oxides of the ring nitrogens are also included, as well as heteroaryls wherein a ring nitrogen is substituted by an optionally substituted alkyl group to form a quaternary amine. Preferred heteroaryl groups are pyridyl, pyrimidyl, thiozolyl, isothiazolyl, oxazolyl, isoxazolyl, quinazolinyl, pyrazinyl and N-oxides thereof and the like. All positional isomers are contemplated (e.g. pyridin-2-yl, pyridin-3-yl, pyridin-4-yl, pyridin-5-yl, pyridin-6-yl, pyrimidin-2-yl, pyrimidin-4-yl, pyrimidin-5-yl and pyrimidin-6-yl). Heteroaryl groups can be optionally substituted as defined herein.
   ◆ The expression "optionally substituted" means not substituted or substituted with one or more substituents simultaneously or independently, chosen from a halogen atom as defined herein; an hydroxyl; a cyano; an azido; a -nitro; a carboxyl; a -CF₃; an alkyl as defined herein; a haloalkyl as defined herein; a fluoroalkyl as defined herein; an alkenyl as defined herein; an alkynyl as defined herein; a cycloalkyl as defined herein; a cycloalkenyl as defined herein; a cycloalkynyl as defined herein; an heterocyclyl as defined herein; an alkoxy as defined herein.
   ◆ The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, preferably hydrochloric acid, and organic acids such as acetic acid, propionic acid, glycolic acid, pyruvic acid, oxylic acid, maleic acid, malonic acid, salicylic acid, succinic acid, fumaric acid, tartaric acid, citric acid, benzoic acid, glutaric acid, cinnamic acid, mandelic acid, malic acid, methanesulfonic acid, ethanesulfonic acid, p-toluenesulfonic acid, benzenesulfonic acid, N-acetylcystein and the like. In addition, these salts may be prepared from addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to, salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyamine resins and the like. The compounds of formula (I) can also be present in the form of zwitterions.
   ◆ Particularly preferred pharmaceutically acceptable salts of compounds of formula (I) are the hydrochloride salts.
   ◆ The compounds of formula (I) can also be solvated, e.g. hydrated. The solvation can be performed in the course of the manufacturing process or can take place e.g. as a consequence of hygroscopic properties of an initially anhydrous compound of formula (I) (hydration). The term "pharmaceutically acceptable salts" also includes physiologically acceptable solvates including water.
   ◆ The mitogen-activated protein kinase kinase inhibitors (MEK inhibitors) as described herein can also be solvated, e.g. hydrated. The solvation can be performed in the course of the manufacturing process (e.g. dimethyl sulfoxide solvate form) or can take place as a consequence of hygroscopic properties of an initially anhydrous MEK inhibitors (e.g. hydration for hydrated form). The term "pharmaceutically acceptable salts" also includes physiologically acceptable solvates including water.
   ◆ "Isomers" are compounds that have identical molecular formulae but that differ in the nature or the sequence of bonding of their atoms or in the arrangement of their atoms in space. Isomers that differ in the arrangement of their atoms in space are termed "stereoisomers". Stereoisomers that are not mirror images of one another are termed "diastereoisomers", and stereoisomers that are non-superimposable mirror images are termed "enantiomers", or sometimes optical isomers.
   ◆ "Prodrug" means a compound that undergoes conversion to the compound of the invention within a biological system. A prodrug is a chemical derivative inactive or less active than the drug itself. After administration and diffusion in the body, the prodrug derivative undergoes one or more metabolic processes that release the active drug. The conversion of the prodrug to the drug is generally carry out under the control of enzymatic processes (usually by metabolic means, e.g. hydrolysis, reduction or oxidation) and less frequently by classical chemical reactions during its diffusion in the body. The linkage between the carrier and the drug can be an, but not limited to, ester, amide, carbonate, carbamate, imine, acetal, ether (e.g. glucoro conjugation), oxydizable function and molecular system, reducible function and reducible molecular system, photoactivated function and photoactivated molecular system. For example, an ester prodrug of a compound containing a hydroxyl group may be convertible by hydrolysis *in vivo* to the parent molecule. Suitable esters of the compounds of the invention containing a hydroxyl group, are for example acetates, citrates, lactates, tartrates, malonates, oxalates, salicylates, propionates, succinates, fumarates, maleates, methylene-bis-*β-*hydroxynaphthoates, gestisates, isethionates, di-p-toluoyltartrates, methanesulphonates, ethanesulphonates, benzenesulphonates, p-toluenesulphonates, cyclohexylsulphamates and quinates. As another example an ester prodrug of the compound of the invention containing a carboxy group may be convertible by hydrolysis *in vivo* to the parent molecule (Examples of ester prodrugs are described by F.J. Leinweber, Drug Metab. Res.1987, (18) pp379, incorporated herein by reference). Similarly, an acyl prodrug of a compound containing an amino group may be convertible by hydrolysis *in vivo* to the parent molecule (examples of prodrugs for these and other functional groups, including amine, alcohol are described in Prodrugs: Challenges and Rewards (Parts 1 and 2); Ed V. Stella, R. Borchardt et al., Springer, 2007, and Prodrugs and Targeted Delivery: Towards Better ADME Properties Ed. J. Rautio, Seies Ed. R. Mannhold, H. Kubinyl, G. Folkers. Wiley-VCH 2011, each of which is incorporated herein by reference).
   ◆ A prodrug carrier system is generally used in order to increase water or lipid solubility, reduce toxicity, increase chemical and biological stability of a sensitive compound, increase the circulating time in the body (T_{*1*/*2*}), increase the total drug exposure (AUC) and organ distribution (PK-PD profiling) and site specific targeting.

Preferred embodiments of the invention are presented hereafter, wherein any combination of two or more of these embodiments is considered within the scope of the present invention.

Namely, the combination of the preferred definitions of each substituent or moiety in formula (I), as defined below, with each other, are within the scope of the present invention.

According to a preferred embodiment, the combination according to the present invention comprises a compound of formula (I), wherein **L₁**, is chosen from a bond or an alkylene;

In a preferred embodiment, the combination as defined above comprises a compound of formula (I), wherein **L₁**, is a bond.

In a preferred embodiment, the combination as defined above comprises a compound of formula (I), wherein **L₁**, is an alkylene.

In another preferred embodiment, the combination as defined above comprises a compound of formula (I), wherein **L₁**, is a carbonyl.

In another preferred embodiment, the combination as defined above comprises a compound of formula (I), wherein **L₁**, is chosen from, -CH₂-, -CHF-, -CF₂-, -CH₂-CH₂.

In another more preferred embodiment, the combination as defined above comprises a compound of formula (I), wherein **L₁**, is -CH₂-.

According to a preferred embodiment, the combination according to the present invention comprises a compound of formula (I), wherein **R₁**, either alone or simultaneously or independently when m > 1, is chosen from a hydrogen atom, a halogen atom, an optionally substituted alkyl, a haloalkyl; a fluoroalkyl; an optionally substituted alkoxy; an azido; a hydroxyl; a cyano; a nitro; -N**R₅R₆**.

In particular, the combination according to the invention comprises a compound of formula (I), wherein **R₁**, either alone or simultaneously or independently when m > 1, is chosen from a hydrogen atom, a halogen atom, an optionally substituted alkyl, a haloalkyl; a fluoroalkyl; an optionally substituted alkoxy; a hydroxyl; -N**R₅R₆**.

Yet, another preferred embodiment provides a combination comprising a compound of formula (I), wherein **R₁**, either alone or simultaneously or independently when m > 1, is chosen from a hydrogen atom, a halogen atom, an optionally substituted alkyl, a haloalkyl; a fluoroalkyl; an optionally substituted alkoxy; a hydroxyl.

In another embodiment, the combination as defined above comprises a compound of formula (I), wherein **R₁**, either alone or simultaneously or independently when m > 1, is chosen from a hydrogen atom, a halogen atom, an optionally substituted alkyl, an optionally substituted alkoxy; a hydroxyl.

In another embodiment, the combination according to the present invention comprises a compound of formula (I), wherein **R₁**, either alone or simultaneously or independently when m > 1, is chosen from a hydrogen atom, a halogen atom, a -N**R₅R₆** wherein **R₅** and **R₆** can be chosen from a hydrogen atom; an optionally substituted alkyl; an optionally substituted cycloalkyl; an optionally substituted heterocyclyl group or **R₅** and **R₆** can be linked together with the nitrogen atom to which they are covalently linked to form an optionally substituted heterocyclyl group.

In an another preferred embodiment, the combination according to the present invention comprises a compound of formula (I), wherein **R₁**, either alone or simultaneously or independently when m > 1, is chosen from a hydrogen atom, a halogen atom, a -N**R₅R₆** wherein **R₅** and **R₆** can be chosen from a hydrogen atom; an optionally substituted alkyl; or **R₅** and **R₆** can be linked together with the nitrogen atom to which they are covalently linked to form an optionally substituted heterocyclyl group.

In another embodiment, the combination according to the present invention comprises a compound of formula (I), wherein **R₁**, either alone or simultaneously or independently when m > 1, is chosen from a hydrogen atom, a halogen atom, a -N**R₅R₆** wherein **R₅** and **R₆** can be chosen from a hydrogen atom; an optionally substituted alkyl.

In particular, the combination according to the present invention comprises a compound of formula (I), wherein **R₁**, either alone or simultaneously or independently when m > 1, is chosen from a hydrogen atom, a halogen atom, a -N**R₅R₆** wherein **R₅** and **R₆** can be chosen from a hydrogen atom; a methyl, an ethyl, an *iso*-propyl, a *tert*-butyl, a cyclopropyl, a cyclobutyl, a cyclopentyl, a cyclohexyl.

In particular, the combination according to the present invention comprises a compound of formula (I), wherein **R₁**, either alone or simultaneously or independently when m > 1, is chosen from a hydrogen atom, a -N**R₅R₆** wherein **R₅** and **R₆** can be chosen from a hydrogen atom; a methyl, an optionally substituted heterocyclyl group.

In particular, the combination according to the present invention comprises a compound of formula (I), wherein **R₁**, either alone or simultaneously or independently when m > 1, is chosen from a hydrogen atom, a -N**R₅R₆** wherein **R₂** and **R₃** can be linked together with the nitrogen atom to which they are covalently linked to form an optionally substituted heterocyclyl group. According to a preferred embodiment, the combination according to the present invention comprises a compound of formula (I), wherein **R₁**, either alone or simultaneously or independently when m > 1, is chosen from a hydrogen atom or a halogen atom.

According to a more preferred embodiment, the combination according to the present invention comprises a compound of formula (I), wherein **R₁**, either alone or simultaneously or independently when m > 1, is chosen from a hydrogen atom, a fluorine, a chlorine, a bromine, more preferably a hydrogen atom or a chlorine.

Yet, according to a more preferred embodiment, the combination according to the present invention comprises a compound of formula (I), wherein **R₁**, either alone or simultaneously or independently when m > 1, is chosen from a hydrogen atom, a fluorine, a chlorine, a bromine, most preferably a hydrogen atom or a fluorine.

According to a preferred embodiment, the combination according to the present invention comprises a compound of formula (I), wherein **R₁**, either alone or simultaneously or independently when m > 1, is chosen from a hydrogen atom, an optionally substituted alkyl, a haloalkyl; a fluoroalkyl.

In a preferred embodiment, the combination according to the present invention comprises a compound of formula (I), wherein **R₁**, either alone or simultaneously or independently when m > 1, is chosen from a hydrogen atom, an alkyl.

In a more preferred embodiment, the combination according to the present invention comprises a compound of formula (I), wherein **R₁**, either alone or simultaneously or independently when m > 1, is chosen from a hydrogen atom, a methyl, an ethyl, a n-propyl, an *iso*-propyl, a n-butyl, an *iso*-butyl, a *sec*-butyl, a *tert*-butyl.

In a still more preferred embodiment, the combination according to the present invention comprises a compound of formula (I), wherein **R₁**, either alone or simultaneously or independently when m > 1, is chosen from a hydrogen atom or a methyl.

In another preferred embodiment, the combination according to the present invention comprises a compound of formula (I), wherein **R₁**, either alone or simultaneously or independently when m > 1, is chosen from a hydrogen atom, a cycloalkyl.

Yet in a more preferred embodiment, the combination according to the present invention comprises a compound of formula (I), wherein **R₁**, either alone or simultaneously or independently when m > 1, is chosen from a hydrogen atom, a cyclopropyl, a cyclobutyl, a cyclopentyl, a cyclohexyl.

Yet, another preferred embodiment provides a combination comprising a compound of formula (I), wherein **R₁**, either alone or simultaneously or independently when m > 1, is chosen from a hydrogen atom, a hydroxyl, an optionally substituted alkoxy.

In a more preferred embodiment, the combination according to the present invention comprises a compound of formula (I), wherein **R₁**, either alone or simultaneously or independently when m > 1, is chosen from a hydrogen atom, a hydroxyl, a methoxy; an ethoxy; a isopropoxy; a *sec*-butoxy, a *tert*-butoxy.

In a further preferred embodiment, the combination according to the present invention comprises a compound of formula (I), wherein **R₁**, either alone or simultaneously or independently when m > 1, is chosen from a hydrogen atom, a hydroxyl, a cyclopropoxy; a cyclobutoxy; a cyclopentoxy; a cyclohexyloxy.

Yet in a more preferred embodiment, the combination according to the present invention comprises a compound of formula (I), wherein **R₁**, either alone or simultaneously or independently when m > 1, is chosen from a hydrogen atom, a chlorine, a hydroxyl, a methoxy. Yet in another more preferred embodiment, the combination according to the present invention comprises a compound of formula (I), wherein **R₁**, either alone or simultaneously or independently when m > 1, is chosen from a hydrogen atom, a chlorine, a hydroxyl.

In another preferred embodiment, the combination according to the invention comprises a compound of formula (I), wherein **R₁**, either alone or simultaneously or independently when m > 1, is chosen from a hydrogen atom, a fluorine, a chlorine, a bromine, a -CF3.

In another preferred embodiment, the combination according to the invention comprises a compound of formula (I), wherein **R₁**, either alone or simultaneously or independently when m > 1, is chosen from a hydrogen atom, a fluorine, a chlorine, a bromine.

In another e preferred embodiment, the combination according to the invention comprises a compound of formula (I), wherein **R₁**, either alone or simultaneously or independently when m > 1, is chosen from a hydrogen atom, a cyano.

According to a preferred embodiment, the combination according to the present invention comprises a compound of formula (I), wherein **R₂** and **R₃** can be, simultaneously or independently, chosen from a hydrogen atom; an optionally substituted alkyl, an optionally substituted alkenyl, an optionally substituted alkynyl, an optionally substituted cycloalkyl, an optionally substituted cycloalkenyl, an optionally substituted cycloalkynyl, an optionally substituted heterocyclyl group.

In another preferred embodiment, the combination according to the invention comprises a compound of formula (I), wherein **R₂** and **R₃** can be, simultaneously or independently, chosen from a hydrogen atom; an optionally substituted alkyl, an optionally substituted cycloalkyl, an optionally substituted heterocyclyl.

In another preferred embodiment, the combination according to the invention comprises a compound of formula (I), wherein **R₂** and **R₃** can be, simultaneously or independently, chosen from a hydrogen atom; an optionally substituted alkyl, an optionally substituted heterocyclyl. In another preferred embodiment, the combination according to the invention comprises a compound of formula (I), wherein **R₂** and **R₃** can be, simultaneously or independently, chosen from a hydrogen atom; an alkyl, a cycloalkyl.

Yet in another preferred embodiment, the combination according to the invention comprises a compound of formula (I), wherein **R₂** and **R₃** can be, simultaneously or independently, chosen from a hydrogen atom or an heterocyclyl.

In a further preferred embodiment, the combination according to the invention comprises a compound of formula (I), wherein **R₂** and **R₃** can be, simultaneously or independently, chosen from a hydrogen atom; an alkyl.

In another preferred embodiment, the combination according to the invention comprises a compound of formula (I), wherein **R₂** and **R₃** can be, simultaneously or independently, chosen from a hydrogen atom; a methyl, an ethyl, a n-propyl, an *iso*-propyl, a n-butyl, an *iso*-butyl, a *sec*-butyl, a *tert*-butyl.

In still more preferred embodiment, the combination according to the invention comprises a compound of formula (I), wherein **R₂** and **R₃** can be, simultaneously or independently, chosen from a hydrogen atom; a *tert*-butyl.

In another preferred embodiment, the combination according to the invention comprises a compound of formula (I), wherein **R₂** and **R₃** can be, simultaneously or independently, chosen from a hydrogen atom; a cyclopropyl, a cyclobutyl, a cyclopentyl, a cyclohexyl.

In another preferred embodiment, the combination according to the invention comprises a compound of formula (I), wherein **R₂** and **R₃** can be, simultaneously or independently, chosen from a hydrogen atom; an heterocyclyl.

In another preferred embodiment, the combination according to the invention comprises a compound of formula (I), wherein **R₂** and **R₃** can be linked together with the nitrogen atom to which they are covalently linked to form an optionally substituted heterocyclyl group.

According to a preferred embodiment, the combination according to the present invention comprises a compound of formula (I), wherein **R₄**, either alone or simultaneously or independently when n > 1, is chosen from a hydrogen atom, a halogen atom, an optionally substituted alkyl, a haloalkyl; a fluoroalkyl; an optionally substituted alkoxy; an azido; a hydroxyl; a cyano; a nitro; -N**R₅R₆**.

In another preferred embodiment, the combination according to the present invention comprises a compound of formula (I), wherein **R₄**, either alone or simultaneously or independently when n > 1, is chosen from a hydrogen atom, a halogen atom, a -N**R₅R₆** wherein **R₅** and **R₆** can be chosen from a hydrogen atom; an optionally substituted alkyl; an optionally substituted cycloalkyl; an optionally substituted heterocyclyl group or **R₅** and **R₆** can be linked together with the nitrogen atom to which they are covalently linked to form an optionally substituted heterocyclyl group.

In another preferred embodiment, the combination according to the present invention comprises a compound of formula (I), wherein **R₄**, either alone or simultaneously or independently when n > 1, is chosen from a hydrogen atom, a -N**R₅R₆** wherein **R₅** and **R₆** can be chosen from a hydrogen atom; an optionally substituted alkyl.

In another preferred embodiment, the combination according to the present invention comprises a compound of formula (I), wherein **R₄**, either alone or simultaneously or independently when n > 1, is chosen from a hydrogen atom, a -N**R₅R₆** wherein **R₅** and **R₆** can be chosen from a hydrogen atom; an optionally substituted cycloalkyl.

In a preferred embodiment, the combination according to the present invention comprises a compound of formula (I), wherein **R₄**, either alone or simultaneously or independently when n > 1, is chosen from a hydrogen atom, a -N**R₅R₆** wherein **R₅** and **R₆** can be chosen from a hydrogen atom; a heterocyclyl group or **R₅** and **R₆** can be linked together with the nitrogen atom to which they are covalently linked to form a heterocyclyl group.

In another preferred embodiment, the combination according to the present invention comprises a compound of formula (I), wherein **R₄**, either alone or simultaneously or independently when n > 1, is chosen from a hydrogen atom, a -N**R₅R₆** wherein **R₅** and **R₆** can be linked together with the nitrogen atom to which they are covalently linked to form a heterocyclyl group.

In a further preferred embodiment, the combination according to the present invention comprises a compound of formula (I), wherein **R₄**, either alone or simultaneously or independently when n > 1, is chosen from a hydrogen atom, a -N**R₅R₆** wherein **R₅** and **R₆** can be chosen from a hydrogen atom or form a heterocyclyl group.

In another preferred embodiment, the combination according to the present invention comprises a compound of formula (I), wherein **R₄**, either alone or simultaneously or independently when n > 1, is chosen from a hydrogen atom, a -N**R₅R₆** wherein **R₅** and **R₆** can be chosen from a hydrogen atom; a methyl, an ethyl, a n-propyl, an *iso*-propyl, a n-butyl, an *iso*-butyl, a *sec*-butyl, a *tert*-butyl.

In another preferred embodiment, the combination according to the present invention comprises a compound of formula (I), wherein **R₄**, either alone or simultaneously or independently when n > 1, is chosen from a hydrogen atom, a -N**R₅R₆** wherein **R₅** and **R₆** can be chosen from a hydrogen atom; a cyclopropyl, a cyclobutyl, a cyclopentyl, a cyclohexyl.

In another preferred embodiment, the combination according to the present invention comprises a compound of formula (I), wherein **R₄**, either alone or simultaneously or independently when n > 1, is chosen from a hydrogen atom, a halogen atom, an optionally substituted alkyl.

In another preferred embodiment, the combination according to the present invention comprises a compound of formula (I), wherein **R₄**, either alone or simultaneously or independently when n > 1, is chosen from a hydrogen atom or a cycloalkyl.

In another preferred embodiment, the combination according to the present invention comprises a compound of formula (I), wherein **R₄**, either alone or simultaneously or independently when n > 1, is chosen from a hydrogen atom, an alkyl.

In another preferred embodiment, the combination according to the present invention comprises a compound of formula (I), wherein **R₄**, either alone or simultaneously or independently when n > 1, is chosen from a hydrogen atom, a methyl, an ethyl, a n-propyl, an *iso*-propyl, a n-butyl, an *iso*-butyl, a *sec*-butyl, a *tert*-butyl.

In another preferred embodiment, the combination according to the present invention comprises a compound of formula (I), wherein **R₄**, either alone or simultaneously or independently when n > 1, is chosen from a hydrogen atom, a halogen atom, an alkyl, a haloalkyl; a fluoroalkyl; an alkoxy, a hydroxyl.

In another more preferred embodiment, the combination according to the present invention comprises a compound of formula (I), wherein **R₄**, either alone or simultaneously or independently when n > 1, is chosen from a hydrogen atom, an alkoxy.

In another preferred embodiment, the combination according to the present invention comprises a compound of formula (I), wherein **R₄**, either alone or simultaneously or independently when n > 1, is chosen from a hydrogen atom, a hydroxyl, a methoxy; a ethoxy; a isopropoxy; a *sec*-butoxy, a *tert*-butoxy.

In a further preferred embodiment, the combination according to the present invention comprises a compound of formula (I), wherein **R₄**, either alone or simultaneously or independently when m > 1, is chosen from a hydrogen atom, a hydroxyl, a cyclopropoxy; a cyclobutoxy; a cyclopentoxy; a cyclohexyloxy.

In a further preferred embodiment, the combination according to the present invention comprises a compound of formula (I), wherein **R₄**, either alone or simultaneously or independently when m > 1, is chosen from a hydrogen atom, a methoxy.

In another erred embodiment, the combination according to the present invention comprises a compound of formula (I), wherein **R₄**, either alone or simultaneously or independently when n > 1, is chosen from a hydrogen atom, a hydroxyl, a methoxy.

In another embodiment, the combination according to the present invention comprises a compound of formula (I), wherein **R₄**, either alone or simultaneously or independently when n > 1, is chosen from a hydrogen atom, a hydroxyl, a methyl, a methoxy.

In another more preferred embodiment, the combination according to the present invention comprises a compound of formula (I), wherein **R₄**, either alone or simultaneously or independently when n > 1, is chosen from a hydrogen atom, a fluorine, a chlorine, a bromine, a -CF3.

In further preferred embodiment, the combination according to the present invention comprises a compound of formula (I), wherein **R₄**, either alone or simultaneously or independently when n > 1, is chosen from a hydrogen atom or a halogen.

In another more preferred embodiment, the combination according to the present invention comprises a compound of formula (I), wherein **R₄**, either alone or simultaneously or independently when n > 1, is chosen from a hydrogen atom, a fluorine, a chlorine.

In another still more preferred embodiment, the combination according to the present invention comprises a compound of formula (I), wherein **R₄**, either alone or simultaneously or independently when n > 1, is chosen from a hydrogen atom or a cyano.

In another more preferred embodiment, the combination according to the present invention comprises a compound of formula (I), wherein **R₄**, either alone or simultaneously or independently when n > 1, is a hydrogen atom.

In another embodiment, the combination according to the invention comprises
◆ a compound chosen from compounds of formula (II), (III), and (IV) as shown below: 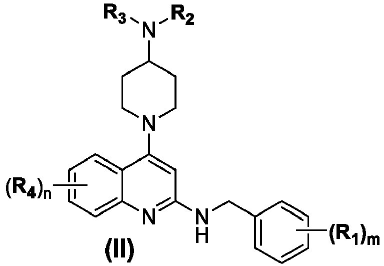 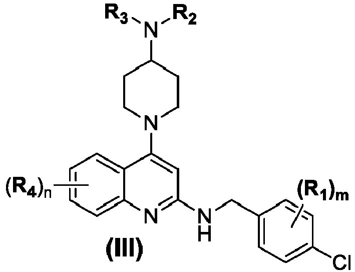 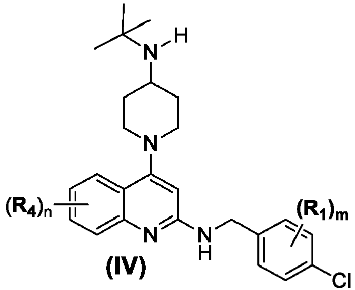 wherein
   - **R₁** , either alone or simultaneously or independently when m > 1 is selected from a hydrogen atom; a halogen atom ; an optionally substituted alkyl; a haloalkyl; a fluoroalkyl; an optionally substituted alkenyl; an optionally substituted alkynyl; an optionally substituted cycloalkyl; an optionally substituted cycloalkenyl; an optionally substituted cycloalkynyl; an optionally substituted alkoxy; -O-**R₇**; an azido; a hydroxyl; a cyano; a nitro; -N**R₅R₆**;
   - **R₂** and **R₃** , simultaneously or independently, are selected from a hydrogen atom; an optionally substituted alkyl; an optionally substituted alkenyl; an optionally substituted alkynyl; an optionally substituted cycloalkyl; an optionally substituted cycloalkenyl; an optionally substituted cycloalkynyl; an optionally substituted heterocyclyl group; or **R₂** and **R₃** can be linked together with the nitrogen atom to which they are covalently linked to form an optionally substituted heterocyclyl group;
   - **R₄** , either alone or simultaneously or independently when n > 1, is selected from a hydrogen atom; a halogen atom; an optionally substituted alkyl; a haloalkyl; a fluoroalkyl; an optionally substituted alkenyl; an optionally substituted alkynyl; an optionally substituted cycloalkyl; an optionally substituted cycloalkenyl; an optionally substituted cycloalkynyl; an optionally substituted alkoxy; -O-**R₇**; an azido; a hydroxyl; a cyano; a nitro; -N**R₅R₆**.
   - **R₅** and **R₆** , simultaneously or independently, are slected from a hydrogen atom; an optionally substituted alkyl; an optionally substituted alkenyl; an optionally substituted alkynyl; an optionally substituted cycloalkyl; an optionally substituted cycloalkenyl; an optionally substituted cycloalkynyl; an optionally substituted heterocyclyl group; or **R₅** and **R₆** can be linked together with the nitrogen atom to which they are covalently linked to form an optionally substituted heterocyclyl group ;
   - **R₇** can be selected from an optionally substituted alkyl; a haloalkyl; a fluoroalkyl; an optionally substituted alkenyl; an optionally substituted alkynyl; an optionally substituted cycloalkyl; an optionally substituted cycloalkenyl; an optionally substituted cycloalkynyl; an optionally substituted heterocyclyl group;
   - n is an integer which can have any one of the values 0, 1, 2, 3 or 4;
   - m is an integer which can have any one of the values 0, 1, 2, 3, 4 or 5 for formula (II); and m is an integer which can have any one of the values 0, 1, 2, 3 or 4 for formulas (III) and (IV);
      and pharmaceutically acceptable salts, hydrates, solvates, prodrugs, polymorphs, tautomers, isotopic variants, isomers, stereoisomers or mixtures of stereoisomers thereof, and
◆ a Mitogen-Activated Protein Kinase Kinase inhibitor (MEK inhibitor) and pharmaceutically acceptable salts, hydrates or solvates thereof.

The compounds of formula (II), (III) and (IV) are preferred embodiments of the compounds of formula (I).

All term definitions and alternative preferred embodiments relating to formula (I), as described above, also apply to formulae (II), (III) and (IV).

In a preferred embodiment, the combination according to the invention comprises a compound chosen from the following compounds:
2-(4-chlorophenylamino)-4-(4-*tert*-butylaminopiperidin-1-yl)-quinoline of formula (**Ia**) (**1-5**) 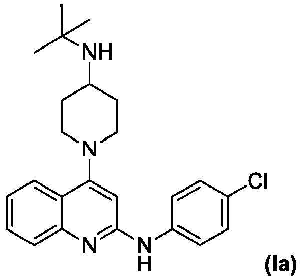 or a pharmaceutically acceptable salt, solvate or prodrug thereof.
2-(4-chlorobenzylamino)-4-(4-*tert*-butylaminopiperidin-1-yl)-quinoline of formula (**Ib**) (**2-2**) 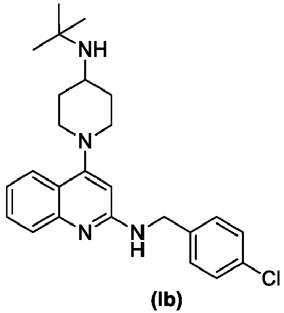 or a pharmaceutically acceptable salt, solvate or prodrug thereof.

In particular, the combination according to the invention comprises a compound chosen from 2-(4-chlorophenylamino)-4-(4-*tert*-butylaminopiperidin-1-yl)-quinoline hydrochloride salt (**1-6**) and 2-(4-chlorobenzylamino)-4-(4-*tert*-butylaminopiperidin-1-yl)-quinoline hydrochloride salt (**2-3**).

The preparation of the compounds of formula (I), (II), (III) and (IV) is described in WO2016/067112 and WO2020/048694. In particular, compounds **1-5, 1-6, 2-2** and **2-3** are described in examples 1 and 2 of WO2016/067112 and WO2020/048694.

In specific embodiments of the invention, the MEK inhibitor can be chosen from the group consisting of Binimetinib [606143-89-9], Cobimetinib [934660-93-2], Selumetinib [606143-52-6], Trametinib [871700-17-3], AZD8330 [869357-68-6], BI-847325 [1207293-36-4], GDC-0623 [1168091-68-6], Mirdametinib (PD0325901) [391210-10-9], PD184352 (CI-1040) [212631-79-3], Pimasertib (AS-703026) [1236699-92-5], Refametinib [923032-37-5], TAK-733 [1035555-63-5], BIX 02188 [1094614-84-2], BIX 02189 [1265916-41-3], Honokiol [35354-74-6], Myricetin [529-44-2], PD98059 [167869-21-8], PD318088 [391210-00-7], SL-327 [305350-87-2], U0126 [109511-58-2].

In further embodiments of the invention, the MEK inhibitor can be chosen from the group consisting of Binimetinib [606143-89-9], Cobimetinib [934660-93-2], Selumetinib [606143-52-6], Trametinib [871700-17-3], AZD8330 [869357-68-6], BI-847325 [1207293-36-4], GDC-0623 [1168091-68-6], Mirdametinib (PD0325901) [391210-10-9], PD184352 (CI-1040) [212631-79-3], Pimasertib (AS-703026) [1236699-92-5], Refametinib [923032-37-5], TAK-733 [1035555-63-5], BIX 02188 [1094614-84-2], BIX 02189 [1265916-41-3], Honokiol [35354-74-6], Myricetin [529-44-2], PD98059 [167869-21-8], PD318088 [391210-00-7], SL-327 [305350-87-2], U0126 [109511-58-2] and pharmaceutically acceptable salts, hydrates or solvates thereof.

In a preferred embodiment, the MEK inhibitor can be chosen from the group consisting of Binimetinib [606143-89-9], Cobimetinib [934660-93-2], Selumetinib [606143-52-6], Trametinib [871700-17-3], AZD8330 [869357-68-6], BI-847325 [1207293-36-4], GDC-0623 [1168091-68-6], Mirdametinib (PD0325901) [391210-10-9], PD184352 (CI-1040) [212631-79-3], Pimasertib (AS-703026) [1236699-92-5], Refametinib [923032-37-5], TAK-733 [1035555-63-5].

In another preferred embodiment, the MEK inhibitor can be chosen from the group consisting of Binimetinib [606143-89-9], Cobimetinib [934660-93-2], Selumetinib [606143-52-6], Trametinib [871700-17-3].

In another preferred embodiment, the MEK inhibitor can be chosen from the group consisting of Binimetinib [606143-89-9], Cobimetinib [934660-93-2], Selumetinib [606143-52-6], Trametinib [871700-17-3] and pharmaceutically acceptable salts, hydrates or solvates thereof.

In a further embodiment, the MEK inhibitor is Trametinib [871700-17-3].

In a preferred embodiment, the MEK inhibitor is a dimethyl sulfoxide solvate form of Trametinib [1187431-43-1].

In particular, the combination according to the invention comprises 2-(4-chlorobenzylamino)-4-(4-*tert*-butylaminopiperidin-1-yl)-quinoline of formula (**Ib**) (**2-2**), or a salt, solvate or prodrug thereof, and Trametinib [871700-17-3] or a pharmaceutically acceptable salt or solvate thereof. In a further embodiment, the combination according to the invention comprises 2-(4-chlorobenzylamino)-4-(4-*tert*-butylaminopiperidin-1-yl)-quinoline of formula (**Ib**) (**2-2**), or a salt, solvate or prodrug thereof, and a dimethyl sulfoxide solvate form of Trametinib [1187431-43-1].

According to one embodiment, the invention relates to a combination consisting of
◆ a compound of formula (I), (II), (III) or (IV) and pharmaceutically acceptable salts, hydrates, solvates, prodrugs, polymorphs, tautomers, isotopic variants, isomers, stereoisomers or mixtures of stereoisomers thereof, and
◆ a Mitogen-Activated Protein Kinase Kinase inhibitor (MEK inhibitor), and pharmaceutically acceptable salts or solvates thereof,
as described above according to all general or specific embodiments.

The invention further relates to a pharmaceutical composition comprising a therapeutically effective amount of a combination as defined above, comprising a compound of formula (I), (II), (III) or (IV) and pharmaceutically acceptable salts, hydrates, solvates, prodrugs, polymorphs, tautomers, isotopic variants, isomers, stereoisomers or mixtures of stereoisomers thereof and a MEK inhibitor and pharmaceutically acceptable salts and solvates thereof, and at least one pharmaceutically acceptable carrier, where each component of the combination can be contained in a separate pharmaceutical composition.

Namely, the components of the combination, which are the active ingredients (compound of formula (I), (II), (III) or (IV) and a MEK inhibitor as defined above) may be administered simultaneously (i.e., concurrently) in either the same or different pharmaceutical compositions, or separately in different pharmaceutical compositions, or sequentially in different pharmaceutical compositions in any order.

Each pharmaceutical composition containing a component of the combination may be administered one or several times, at one or different dosages, with the same or a different route of administration.

The amounts of the active ingredient(s), the relative timings and the routes of administration will be selected in order to achieve the desired combined therapeutic effect.

According to a preferred embodiment, each component of the combination can be contained in a separate pharmaceutical composition.

All general and preferred embodiments described above with reference to the combination of a compound of formula (I), (II), (III) or (IV) and a MEK inhibitor, and each component of this combination, also apply to the pharmaceutical compositions.

The pharmaceutical composition of the invention can be suitable for oral, parenteral, ocular, transdermal, or nasal administration, or for inhalation.

The term "pharmaceutically acceptable carrier" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, uses thereof in the compositions of the invention are contemplated. Supplementary active compounds can also be incorporated into the compositions. These compositions can be prepared by applying known techniques in the art such as described *in* Ansel's Pharmaceutical Dosage Forms and Drug Delivery Systems (Tenth Edition) 2014, Edited by Loyd Allen, Howard C. Ansel, published by Wolters Kluwer Health and Remington: The Science and Pratice of Pharmacy (Twenty-second Edition) 2012, Edited by Loyd V. Allen, Published by Pharmaceutical Press.

The invention further relates to a combination comprising a compound of formula (I) or a pharmaceutically acceptable salt or solvate thereof and a MEK inhibitor or a pharmaceutically acceptable salt or solvate thereof, as defined above, for use in the treatment and/or prevention and/or decrease of liver cancers related diseases, in particular hepatocarcinoma, hepatoblastoma or cholangiocarcinoma.

The invention further relates to a combination comprising a compound of formula (II), (III) or (IV) or a pharmaceutically acceptable salt or solvate thereof and a MEK inhibitor or a pharmaceutically acceptable salt or solvate thereof, as defined above, for use in the treatment and/or prevention and/or decrease of liver cancers related diseases, in particular hepatocarcinoma, hepatoblastoma or cholangiocarcinoma.

The invention also relates to a combination comprising a compound of formula (II), (III) or (IV) or a pharmaceutically acceptable salt or solvate thereof and a MEK inhibitor or a pharmaceutically acceptable salt or solvate thereof, as defined above, for use in the treatment and/or prevention and/or decrease of liver cancers related diseases, in particular intrahepatic cholangiocarcinoma and/or extrahepatic cholangiocarcinoma.

The invention further relates to a combination comprising a compound of formula (I) chosen from 2-(4-chlorophenylamino)-4-(4-*tert*-butylaminopiperidin-1-yl)-quinoline (**1-5**) and 2-(4-chlorobenzylamino)-4-(4-*tert*-butylaminopiperidin-1-yl)-quinoline (**2-2**) and a MEK inhibitor, as defined above, for use in the treatment and/or prevention and/or decrease of liver cancers related diseases, in particular hepatocarcinoma, hepatoblastoma or cholangiocarcinoma.

The invention further relates to a combination comprising a compound of formula (I) chosen from 2-(4-chlorophenylamino)-4-(4-*tert*-butylaminopiperidin-1-yl)-quinoline hydrochloride salt (**1-6**) and 2-(4-chlorobenzylamino)-4-(4-*tert*-butylaminopiperidin-1-yl)-quinoline hydrochloride salt (**2-3**) and a MEK inhibitor, as defined above, for use in the treatment and/or prevention and/or decrease of liver cancers related diseases, in particular hepatocarcinoma, hepatoblastoma or cholangiocarcinoma.

The invention also relates to a combination comprising a compound of formula (I) chosen from 2-(4-chlorophenylamino)-4-(4-*tert*-butylaminopiperidin-1-yl)-quinoline (**1-5**) and 2-(4-chlorobenzylamino)-4-(4-*tert*-butylaminopiperidin-1-yl)-quinoline (**2-2**) and a MEK inhibitor, as defined above, for use in the treatment and/or prevention and/or decrease of liver cancers related diseases, in particular intrahepatic cholangiocarcinoma and/or extrahepatic cholangiocarcinoma.

The invention also relates to a combination comprising a compound of formula (I) chosen from 2-(4-chlorophenylamino)-4-(4-*tert*-butylaminopiperidin-1-yl)-quinoline hydrochloride salt (**1-6**) and 2-(4-chlorobenzylamino)-4-(4-*tert*-butylaminopiperidin-1-yl)-quinoline hydrochloride salt (**2-3**) and a MEK inhibitor, as defined above, for use in the treatment and/or prevention and/or decrease of liver cancers related diseases, in particular intrahepatic cholangiocarcinoma and/or extrahepatic cholangiocarcinoma.

In another aspect, the invention relates to a combination comprising a compound of formula (I) chosen from 2-(4-chlorophenylamino)-4-(4-*tert*-butylaminopiperidin-1-yl)-quinoline (**1-5**) and 2-(4-chlorobenzylamino)-4-(4-*tert*-butylaminopiperidin-1-yl)-quinoline (**2-2**) and a MEK inhibitor, as defined above, for use in the treatment and/or prevention and/or decrease of liver cancers related diseases, in particular hepatocarcinoma.

In a further aspect, the invention relates to a combination comprising a compound of formula (I) chosen from 2-(4-chlorophenylamino)-4-(4-*tert*-butylaminopiperidin-1-yl)-quinoline hydrochloride salt (**1-6**) and 2-(4-chlorobenzylamino)-4-(4-*tert*-butylaminopiperidin-1-yl)-quinoline hydrochloride salt (**2-3**) and a MEK inhibitor, as defined above, for use in the treatment and/or prevention and/or decrease of liver cancers related diseases, in particular hepatocarcinoma.

In a further aspect, the invention relates to a combination of 2-(4-chlorobenzylamino)-4-(4-*tert-*butylaminopiperidin-1-yl)-quinoline of formula (**Ib**) (**2-2**) or a pharmaceutically acceptable salt, solvate or prodrug thereof and Trametinib [871700-17-3] or a pharmaceutically acceptable salt or solvate thereof, for use in the treatment and/or prevention and/or decrease of liver cancers related diseases, in particular hepatocarcinoma, hepatoblastoma or cholangiocarcinoma.

In further preferred aspect, the invention relates to a combination of 2-(4-chlorobenzylamino)-4-(4-*tert*-butylaminopiperidin-1-yl)-quinoline of formula (**Ib**) (**2-2**) or a pharmaceutically acceptable salt, solvate or prodrug thereof and Trametinib [871700-17-3] or a pharmaceutically acceptable salt or solvate thereof, for use in the treatment and/or prevention and/or decrease of hepatocarcinoma.

In further preferred aspect, the invention relates to a combination of 2-(4-chlorobenzylamino)-4-(4-*tert*-butylaminopiperidin-1-yl)-quinoline of formula (**Ib**) (**2-2**) or a pharmaceutically acceptable salt, solvate or prodrug thereof and Trametinib [871700-17-3] or a pharmaceutically acceptable salt or solvate thereof, for use in the treatment and/or prevention and/or decrease of cholangiocarcinoma wherein the cholangiocarcinoma is a intrahepatic cholangiocarcinoma or a extrahepatic cholangiocarcinoma.

In further aspect, the invention relates to a combination of 2-(4-chlorobenzylamino)-4-(4-*tert-*butylaminopiperidin-1-yl)-quinoline of formula (**Ib**) (**2-2**) or a pharmaceutically acceptable salt, solvate or prodrug thereof and the dimethyl sulfoxide (DMSO) solvate form 1:1 of Trametinib [1187431-43-1], for use in the treatment and/or prevention and/or decrease of liver cancers related diseases, in particular hepatocarcinoma, hepatoblastoma or cholangiocarcinoma.

In further preferred aspect, the invention relates to a combination of 2-(4-chlorobenzylamino)-4-(4-*tert*-butylaminopiperidin-1-yl)-quinoline of formula (**Ib**) (**2-2**) or a pharmaceutically acceptable salt, solvate or prodrug thereof and the dimethyl sulfoxide (DMSO) solvate form 1:1 of Trametinib [1187431-43-1], for use in the treatment and/or prevention and/or decrease of hepatocarcinoma.

In further preferred aspect, the invention relates to a combination of 2-(4-chlorobenzylamino)-4-(4-*tert*-butylaminopiperidin-1-yl)-quinoline of formula (**Ib**) (**2-2**) or a pharmaceutically acceptable salt, solvate or prodrug thereof and the dimethyl sulfoxide (DMSO) solvate form 1:1 of Trametinib [1187431-43-1], for use in the treatment and/or prevention and/or decrease of cholangiocarcinoma wherein the cholangiocarcinoma is a intrahepatic cholangiocarcinoma or a extrahepatic cholangiocarcinoma.

More preferably, the combination comprises 2-(4-chlorobenzylamino)-4-(4-*tert-*butylaminopiperidin-1-yl)-quinoline of formula (**Ib**) (**2-2**), or a pharmaceutically acceptable salt, solvate or prodrug thereof, and Trametinib [871700-17-3], or a pharmaceutically acceptable salt or solvate thereof.

In a further aspect, the combination comprises 2-(4-chlorobenzylamino)-4-(4-*tert-*butylaminopiperidin-1-yl)-quinoline of formula (**Ib**) (**2-2**), or a pharmaceutically acceptable salt, solvate or prodrug thereof, and the dimethyl sulfoxide (DMSO) solvate form 1:1 of Trametinib [1187431-43-1].

In a preferred embodiment, the liver cancer related diseases is a cholangiocarcinoma.

In a more preferred embodiment, the liver cancer related diseases is selected from an intrahepatic cholangiocarcinoma and/or an extrahepatic cholangiocarcinoma.

In a further preferred embodiment, the liver cancer related diseases is a hepatocarcinoma.

In a further preferred embodiment, the liver cancer related diseases is a hepatoblastoma

According to the invention, each component of the combination can be for simultaneous, separate or sequential use in liver cancer therapy.

According to the invention, the compound of formula (I), (II), (III) or (IV) as defined above and the MEK inhibitor as defined above, as well as the pharmaceutical compositions containing them, wherein each component of the composition can be comprised in a separate pharmaceutical composition, can be administered to a human being or an animal in need thereof as a combination therapy in the treatment and/or the decreasing and/or prevention of a liver cancer, wherein said administration can be simultaneous, separate or sequential.

All general and preferred embodiments described above with reference to the combination of a compound of formula (I), (II), (III) or (IV) and a MEK inhibitor, and to each component of this combination, also apply to their use in the treatment and/or prevention and/or decrease of liver cancers related diseases, in particular hepatocarcinoma, hepatoblastoma or cholangiocarcinoma.

In another aspect, the present invention also relates to a method of treatment and/or decreasing and/or prevention of liver cancers related diseases, in particular hepatocarcinoma, hepatoblastoma or cholangiocarcinoma, comprising administering to a patient in need thereof a combination comprising a compound of formula (I) (II), (III) or (IV) or a pharmaceutically acceptable salt or solvate thereof and a MEK inhibitor or a pharmaceutically acceptable salt or solvate thereof, as described above.

In another aspect, the present invention also relates to a method of treatment and/or decreasing and/or prevention of liver cancers related diseases, in particular a cholangiocarcinoma wherein the cholangiocarcinoma is an intrahepatic cholangiocarcinoma or an extrahepatic cholangiocarcinoma.

All general and preferred embodiments described above with reference to the combination of a compound of formula (I), (II), (III) or (IV) and a MEK inhibitor, and to each component of this combination, also apply to the method of treatment and/or decreasing and/or prevention of liver cancers related diseases, in particular hepatocarcinoma, hepatoblastoma or cholangiocarcinoma.

As used herein, the terms "subject" or "patient" are used interchangeably. As used herein, the terms "subject" and "subjects" refer to an animal (e.g., birds, reptiles, and mammals), preferably a mammal including a non-primate (e.g., a camel, donkey, zebra, cow, pig, horse, goat, sheep, cat, dog, rat, and mouse) and a primate (e.g., a monkey, chimpanzee, and a human), and most preferably a human.

As used herein, the terms "therapies" and "therapy" can refer to any protocol(s), method(s), compositions, formulations, and/or agent(s) that can be used in the prevention, treatment, management, or amelioration of a disease. In certain embodiments, the terms "therapies" and "therapy" refer to biological therapy, supportive therapy, and/or other therapies useful in treatment, management, prevention, or amelioration of the different diseases known to one of skill in the art.

The term "cholangiocarcinoma" refers to cancer that forms in the bile ducts, which are a series of thin tubes that go from the liver to the small intestine. Bile ducts carry a fluid called bile from the liver and gallbladder into the small intestine, where it helps digest the fats in food. Cholangiocarcinoma is also known as bile duct cancer

The term "intrahepatic cholangiocarcinoma" refers to a cholangiocarcinoma which occurs in the parts of the bile ducts within the liver

The term "extrahepatic cholangiocarcinoma" refers to a cholangiocarcinoma which occurs in the parts of the bile ducts nearest the small intestine.

The term "a therapeutically effective amount" of a compound means an amount of compound that is effective to prevent, alleviate or ameliorate symptoms of disease or prolong the survival of the subject being treated. Determination of a therapeutically effective amount is within the skill in the art. The therapeutically effective amount or dosage of a compound according to this invention can vary within wide limits and may be determined in a manner known in the art. Such dosage will be adjusted to the individual requirements in each particular case including the specific compound(s) being administered, the route of administration, the condition being treated, as well as the patient being treated. In general, in the case of oral or parenteral administration to adult humans weighing approximately 70 kg, a daily dosage of:
- from 50 mg to 500 mg, preferably from 50 mg to 200 mg, more preferably from 50 mg to 100 mg, as regards the compound of formula (I), (II), (III) or (IV), or a pharmaceutically acceptable salt or solvate thereof,
   and
- from 0.5 mg to 150 mg, preferably from 1 mg to 100 mg, more preferably from 1.5 mg to 90 mg, as regards the MEK inhibitor or a pharmaceutically acceptable salt or solvate thereof, should be appropriate,
   although the upper limit can be exceeded when indicated,
- or, as regards the MEK inhibitor Trametinib or a pharmaceutically acceptable salt or solvate thereof from 0.5 mg to 5 mg, preferably from 0.5 mg to 2 mg, more preferably from 1 mg to 2 mg, still more preferably from 1.5 mg to 2 mg, should be appropriate, although the upper limit can be exceeded when indicated.

The daily dosage can be administered as a single dose or in divided doses, or for parenteral administration, it can be given as continuous infusion.

The invention further relates to a kit comprising the combination of a compound of formula (I), (II), (III) or (IV) or a pharmaceutically acceptable salt or solvate thereof and a MEK inhibitor or a pharmaceutically acceptable salt or solvate thereof, as defined above, in which both or either of the components are/is in the form of a pharmaceutical composition which may be administered simultaneously, separately or sequentially.

### Examples

### ▪ Example 1: In vitro evaluation of the cytotoxicity activity of compound 2-2, Hydroxychloroquine (HCQ) and Trametinib in intrahepatic cholangiocarcinoma cell line HuCCT1 (KRAS G12D status)

Cell culture: HuCCT1 cell line harboring KRAS G12D mutation (JCRB Cell Bank #JCRB0425) was maintained in RPMI medium (Dutscher #L0498-500) containing 1% penicillin streptomycin (Dutscher #P06-07100) and 10% fetal bovine serum (Dutscher #SV30160.03C) and were cultured at 37°C with 5% CO₂ in humidified cells incubator.

HuCCT1 cells were plated at 4.10³ cells per well in Greiner Bio-one µClear^{®} P96 well plates (Dutscher #655098) with 80 µL of culture media. After 24 hours, HuCCT1 cells were treated with compounds **2-2,** hydroxychloroquine (HCQ) or Trametinib (Selleckchem #S2673). Cell viability was assessed 72 hours post molecular treatment by CellTiter-Glo^{®} luminescent cell viability assay (Promega #G7573) according to the manufacturer protocol and using an Infinity F200 Pro luminometer (Tecan). For compounds **2-2** and Trametinib, DMSO was used as negative control and for HCQ, water was used as negative control. IC₅₀ values were determined as the dose of compound required to reduce luminescent values to 50% of the reference signal obtained for untreated cell cultures. Raw data were analyzed using GraphPad Prism Software v9.4 (GraphPad Software, Inc. La Jolla, CA). For all experiments, analyzed data are shown as mean of triplicate and four independent experiments. The three compounds **2-2,** HCQ and Trametinib demonstrated a dose-response cytotoxic activity against intrahepatic cholangiocarcinoma cell line HuCCT1 (Table 1).

**Table 1: Cell Viability assay (IC₅₀, µM) of compound 2-2, hydroxychloroquine (HCQ) and Trametinib on intrahepatic cholangiocarcima cell line HuCCT1**

| | **2-2** | **Hydroxychloroquine** | **Trametinib** |
|---|---|---|---|
| **IC₅₀ (µM ± SD)** | 2.2 ± 0.2 | 27.2 ± 1.2 | 0.31 ± 0.26 |

### ▪ Example 2: In vitro evaluation of the cytotoxicity activity combination effect of the binary drug-drug association of compound 2-2 + Trametinib and hydroxychloroquine (HCQ) + Trametinib in intrahepatic cholangiocarcinoma cell line HuCCT1 (KRAS G12D status)

The HuCCT1 cell viability assay with binary drug-drug association was performed as described in example 1. The drug-drug binary associations of compounds **2-2** + Trametinib and HCQ + Trametinib were tested in a checkerboard fashion in Greiner Bio-one µClear^{®} 96-well plates (Dutscher #655098). The concentration ranges were selected nearby and surrounding the IC₅₀ values of each individual compound. Compound **2-2** in combination with Trametinib was tested at 1.28 µM, 1.6 µM, 2.0 µM, 2.5 µM and 3.125 µM. HCQ in combination with Trametinib was tested at 15 µM, 20 µM, 30 µM, 45 µM, 60 µM. Trametinib in combination with compound **2-2** or in combination with HCQ was tested in both cases at 0.005 µM, 0.03 µM, 6.0 µM, 15 µM, 30 µM, 60 µM, 90 µM. The combination effects of both binary drug-drug association compound **2-2** + Trametinib and HCQ + Trametinib were analyzed with two softwares: MacSynergy^{™} II (1) and SynergyFinder (2). MacSynergy^{™} II was used to analyze the combination effect according to the Bliss independence model which assumes probabilistic statistical independence between the combined compounds.(3) SynergyFinder was used to analyze the combination effect according to the Loewe additivity model which is based on its principle of a sham drug-drug combination which assumes no interaction when a compound is combined with itself (4).

### Combination effect analysis according to the Loewe additivity model (SynergyFinder):

The combination effects of the drug-drug associations of compound **2-2** + Trametinib (Table 2) and HCQ + Trametinib (Table 3) were evaluated according to the Loewe additivity model using SynergyFinder for their respective capacities to inhibit the growth of intra-hepatic cholangiocarcinoma cell line HuCCT1.

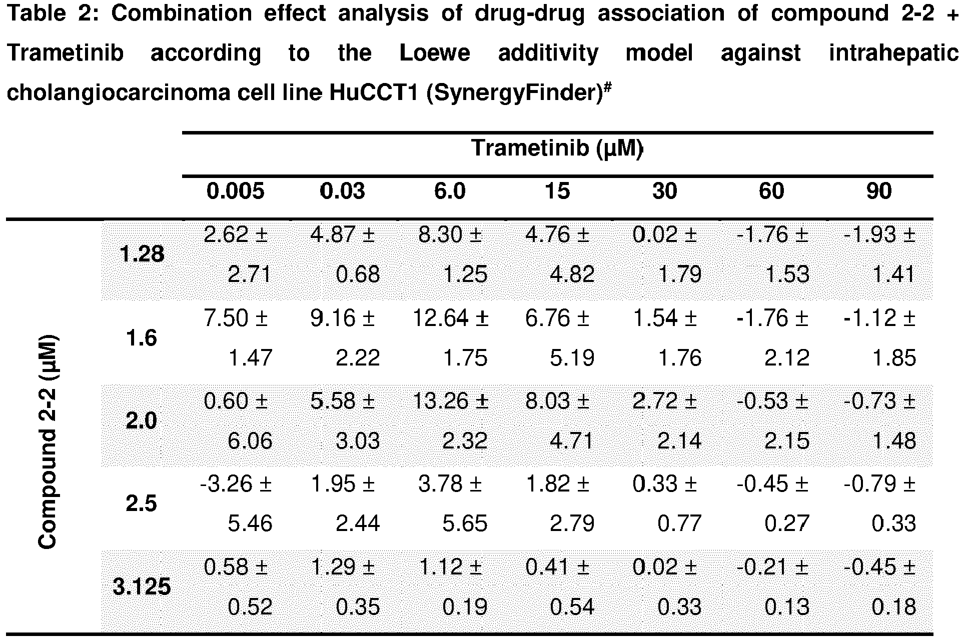

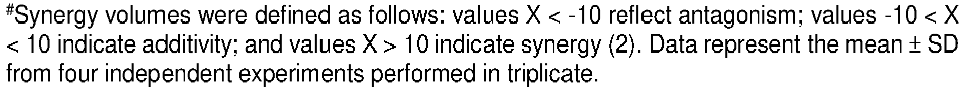

The drug-drug association of compound **2-2** + Trametinib showed an overall additive combination effect according to the Loewe additivity model for their properties to inhibit the growth of intrahepatic cholangiocarcinoma cell line HuCCT1 by inducing a cytotoxic effect evaluated by the CellTiter-Glo^{®} luminescent cell viability assay (Table 2). The overall additive combination effect, of the drug-drug association of compound **2-2** + Trametinib, according to the Loewe non-independence additivity model is not a matter of substantive disagreement with the respective and distinct mode of action of both compounds, namely compound **2-2** as an autophagy inhibitor and Trametinib as a MEK inhibitor.

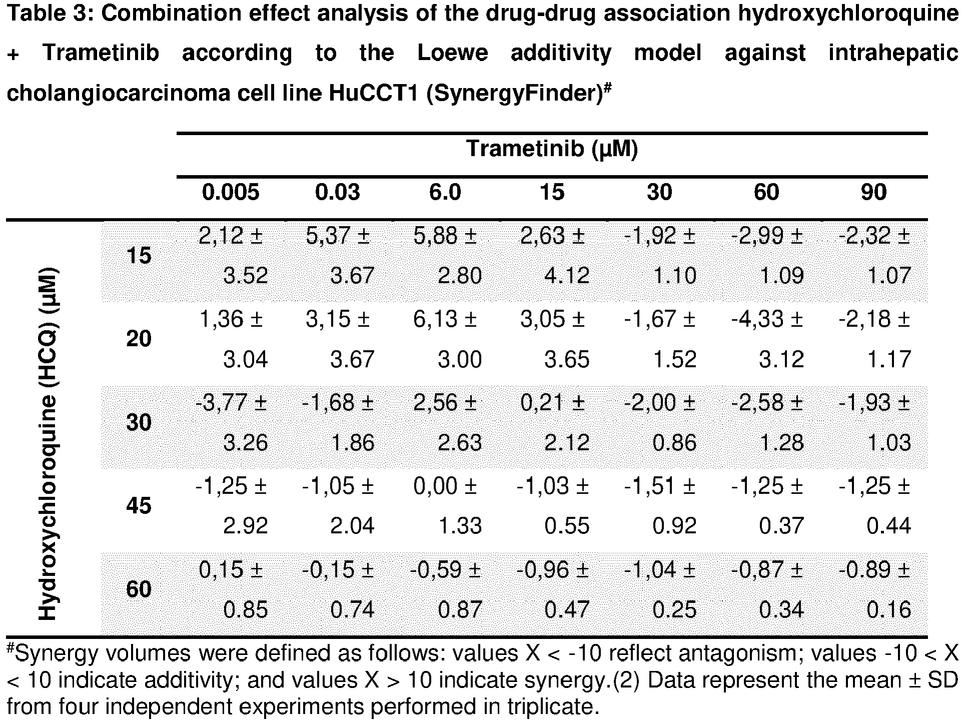

The drug-drug association of hydroxychloroquine + Trametinib showed an overall additive combination effect according to the Loewe non-independence model, for their properties to inhibit the growth of intrahepatic cholangiocarcima cell line HuCCT1 by inducing a cytotoxic effect evaluated by the CellTiter-Glo^{®} luminescent cell viability assay (Table 3). The overall additive combination effect, of the drug-drug association of hydroxychloroquine + Trametinib, according to the Loewe non-independence additivity model is not a matter of substantive disagreement with the respective and distinct mode of action of both compounds, namely hydroxychloroquine as an autophagy inhibitor and Trametinib as a MEK inhibitor.

### Combination effect analysis according to the Bliss independence model (MacSynergy^{™} II):

The combination effects of the drug-drug associations of compound **2-2** + Trametinib and hydroxychloroquine + Trametinib were evaluated according to the Bliss independence model using MacSynergy^{™} II for their respective capacities to inhibit the growth of intrahepatic cholangiocarcinoma cell line HuCCT1 (Table 4).

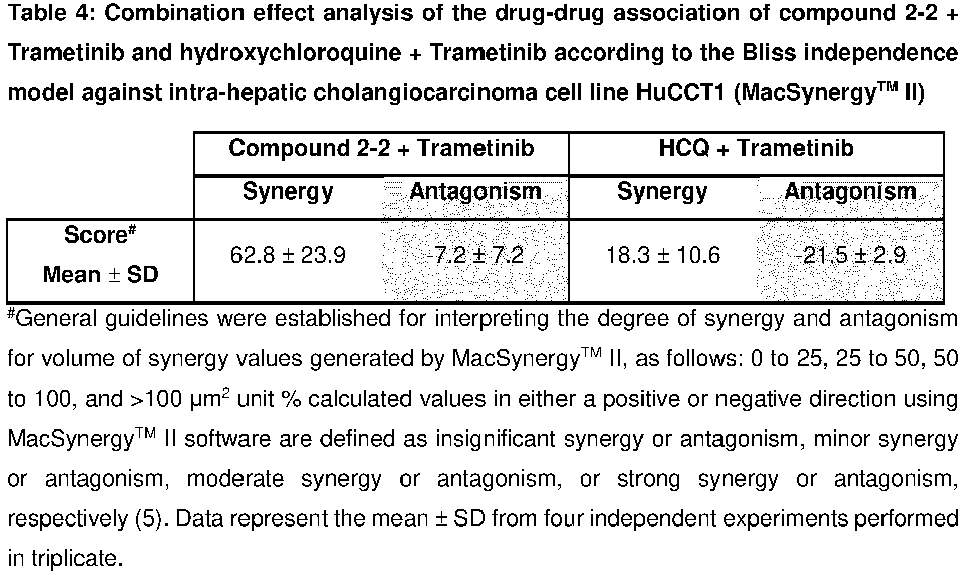

According to the Bliss independence model, the drug-drug association of compound **2-2** + Trametinib showed significant moderate synergy in inhibiting the growth of intra-hepatic cholangiocarcinoma cell line HuCCT1 with a potential significant impact *in vivo* and in clinical setting. In comparison, the drug-drug association of hydroxychloroquine + Trametinib showed insignificant synergy drug-drug combination effect according to the Bliss independence model with an overall additive effect to inhibit the growth of intra-hepatic cholangiocarcinoma cell line HuCCT1 (5, 6).

Both molecules, compound **2-2** and hydroxychloroquine are well known from the art as autophagy inhibitors.(7, 8) However, when both these autophagy inhibitors are each individually associated with Trametinib, a MEK inhibitor, to inhibit the growth of intra-hepatic cholangiocarcinoma HuCCT1 cell line, hydroxychloroquine only showed an additive effect, while compound **2-2** showed a significant synergy combination effect.

### Bibliography

1. Prichard, M. N., and Shipman, C. (1990) A three-dimensional model to analyze drug-drug interactions. Antiviral Res. 14, 181-205
2. Zheng, S., Wang, W., Aldahdooh, J., Malyutina, A., Shadbahr, T., Tanoli, Z., Pessia, A., and Tang, J. (2022) SynergyFinder Plus: Toward Better Interpretation and Annotation of Drug Combination Screening Datasets. Genomics. Proteomics Bioinformatics. 10.1016/J.GPB.2022.01.004
3. Bliss, C. I. (1939) THE TOXICITY OF POISONS APPLIED JOINTLY1. Ann. Appl. Biol. 26, 585-615
4. Loewe, S. (1953) The problem of synergism and antagonism of combined drugs. Arzneimittelforschung. 3, 285-290
5. Smee, D. F., and Prichard, M. N. (2017) Comparison of three dimensional synergistic analyses of percentage versus logarithmic data in antiviral studies. Antiviral Res. 145, 1-5
6. Prichard, M. N., and Shipman Jr., C. (1990) A three-dimensional model to analyze drug-drug interactions. Antivir. Res. 14, 181-205
7. HALFON, P., BASSISSI, F., BRUN, S., COURCAMBECK, J., and RACHID, M. (2020) SUBSTITUTED 2,4 DIAMINO-QUINOLINE AS NEW MEDICAMENT FOR FIBROSIS, AUTOPHAGY AND CATHEPSINS B (CTSB), L (CTSL) AND D (CTSD) RELATED DISEASES. WO2020048694
8. Pasquier, B. (2016) Autophagy inhibitors. Cell. Mol. Life Sci. 73, 985-1001

## Claims

1. A combination comprising
◆ a compound of formula (I) 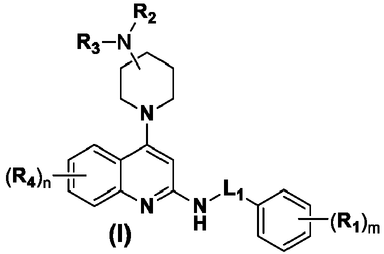 wherein
• **L₁** is chosen from a single bond; an optionally substituted (CH₂)p group; an optionally substituted alkylene; a carbonyl.
• **R₁** , either alone or simultaneously or independently when m > 1 is selected from a hydrogen atom; a halogen atom ; an optionally substituted alkyl; a haloalkyl; a fluoroalkyl; an optionally substituted alkenyl; an optionally substituted alkynyl; an optionally substituted cycloalkyl; an optionally substituted cycloalkenyl; an optionally substituted cycloalkynyl; an optionally substituted alkoxy; -O-**R₇**; -O-(CO)-**R₇**; -O-(CO)-N**R₅R₆**; -N**R₅**-(CO)-**R₇**; -O-(CO)-O**R₇**; -N**R₅**-(CO)-O-**R₇**; an azido; a hydroxyl; a cyano; a nitro; -N**R₅R₆**;
• **R₂** and **R₃** , simultaneously or independently, are selected from a hydrogen atom; an optionally substituted alkyl; an optionally substituted alkenyl; an optionally substituted alkynyl; an optionally substituted cycloalkyl; an optionally substituted cycloalkenyl; an optionally substituted cycloalkynyl; an optionally substituted heterocyclyl group; an optionally substituted aryl; an optionally substituted benzyl; an optionally substituted heteroaryl;
or **R₂** and **R₃** can be linked together with the nitrogen atom to which they are covalently linked to form an optionally substituted heterocyclyl group;
• **R₄** , either alone or simultaneously or independently when n > 1, is selected from a hydrogen atom; a halogen atom; an optionally substituted alkyl; a haloalkyl; a fluoroalkyl; an optionally substituted alkenyl; an optionally substituted alkynyl; an optionally substituted cycloalkyl; an optionally substituted cycloalkenyl; an optionally substituted cycloalkynyl; an optionally substituted alkoxy; -O-**R₇**; -O-(CO)-**R₇**; -O-(CO)-N**R₅R₆**; -N**R₅**-(CO)-**R₇**; -O-(CO)-O**R₇**; -N**R₅**-(CO)-O-**R₇**; an azido; a hydroxyl; a cyano; a nitro; -N**R₅R₆**.
• **R₅** and **R₆** , simultaneously or independently, are slected from a hydrogen atom; an optionally substituted alkyl; an optionally substituted alkenyl; an optionally substituted alkynyl; an optionally substituted cycloalkyl; an optionally substituted cycloalkenyl; an optionally substituted cycloalkynyl; an optionally substituted heterocyclyl group; an optionally substituted aryl; an optionally substituted heteroaryl ;
or **R₅** and **R₆** can be linked together with the nitrogen atom to which they are covalently linked to form an optionally substituted heterocyclyl group ;
• **R₇** can be selected from an optionally substituted alkyl; a haloalkyl; a fluoroalkyl; an optionally substituted alkenyl; an optionally substituted alkynyl; an optionally substituted cycloalkyl; an optionally substituted cycloalkenyl; an optionally substituted cycloalkynyl; an optionally substituted heterocyclyl group; an optionally substituted aryl; an optionally substituted heteroaryl;
• n is an integer which can have any one of the values 0, 1, 2, 3 or 4;
• m is an integer which can have any one of the values 0, 1, 2, 3, 4 or 5;
• p is an integer which can have any one of the values 0 or 1;
and pharmaceutically acceptable salts, hydrates, solvate, prodrugs, polymorphs, tautomers, isotopic variants, isomers, stereoisomers or mixtures of stereoisomers thereof, and
◆ a Mitogen-Activated Protein Kinase Kinase inhibitor (MEK inhibitor), and pharmaceutically acceptable salts or solvates thereof.

2. The combination according to claim 1, comprising a compound of formula (I) wherein **R₁**, either alone or simultaneously or independently when m>1, is chosen from a hydrogen atom, a halogen atom, an optionally substituted alkyl, a haloalkyl; a fluoroalkyl; an optionally substituted alkoxy; a hydroxyl.

3. The combination according to claim 1 or 2, comprising a compound of formula (I) wherein **R₁**, either alone or simultaneously or independently when m>1, is chosen from a hydrogen atom, a fluorine, a chlorine or a bromine.

4. The combination according to any one of claims 1 to 3, comprising a compound of formula (I) wherein **R₂** and **R₃** , simultaneously or independently, are selected from a hydrogen atom; an optionally substituted alkyl, an optionally substituted cycloalkyl, an optionally substituted heterocyclyl.

5. The combination according to any one of claims 1 to 4, comprising a compound of formula (I) wherein **R₂** and **R₃** , simultaneously or independently, are selected from a hydrogen atom; a cyclopropyl, a cyclobutyl, a cyclopentyl, a cyclohexyl.

6. The combination according to any one of claims 1 to 4, comprising a compound of formula (I) wherein **R₂** and **R₃** can be linked together with the nitrogen atom to which they are covalently linked to form an optionally substituted heterocyclyl group.

7. The combination according to any one of claims 1 to 6, comprising a compound of formula (I) wherein **R₄**, either alone or simultaneously or independently when n > 1, is chosen from a hydrogen atom, a halogen atom, an alkyl, a haloalkyl; a fluoroalkyl; an alkoxy, a hydroxyl.

8. The combination according to any one of claims 1 to 7, comprising a compound of formula (I) wherein **R₄**, either alone or simultaneously or independently when n > 1, is chosen from a hydrogen atom, a hydroxyl, a methoxy; a ethoxy; a isopropoxy; a sec-butoxy, a *tert-*butoxy.

9. The combination according to any one of claims 1 to 8, in which the compound of formula (I) is chosen from compounds of formula (II), (III), and (IV) below: 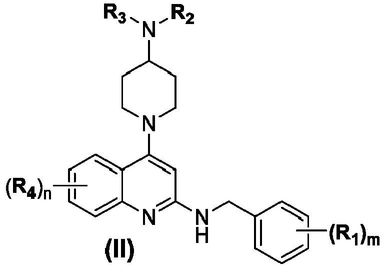 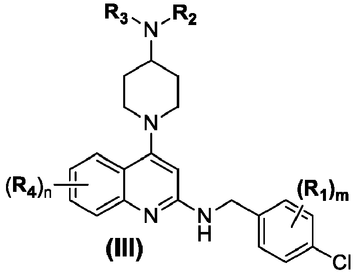 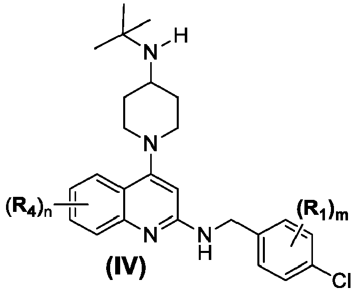 wherein
• **R₁** , either alone or simultaneously or independently when m > 1 is selected from a hydrogen atom; a halogen atom ; an optionally substituted alkyl; a haloalkyl; a fluoroalkyl; an optionally substituted alkenyl; an optionally substituted alkynyl; an optionally substituted cycloalkyl; an optionally substituted cycloalkenyl; an optionally substituted cycloalkynyl; an optionally substituted alkoxy; -O-**R₇**; an azido; a hydroxyl; a cyano; a nitro; -N**R₅R₆**;
• **R₂** and **R₃** , simultaneously or independently, are selected from a hydrogen atom; an optionally substituted alkyl; an optionally substituted alkenyl; an optionally substituted alkynyl; an optionally substituted cycloalkyl; an optionally substituted cycloalkenyl; an optionally substituted cycloalkynyl; an optionally substituted heterocyclyl group; or **R₂** and **R₃** can be linked together with the nitrogen atom to which they are covalently linked to form an optionally substituted heterocyclyl group;
• **R₄** , either alone or simultaneously or independently when n > 1, is selected from a hydrogen atom; a halogen atom; an optionally substituted alkyl; a haloalkyl; a fluoroalkyl; an optionally substituted alkenyl; an optionally substituted alkynyl; an optionally substituted cycloalkyl; an optionally substituted cycloalkenyl; an optionally substituted cycloalkynyl; an optionally substituted alkoxy; -O-**R₇**; an azido; a hydroxyl; a cyano; a nitro; -N**R₅R₆**.
• **R₅** and **R₆** , simultaneously or independently, are slected from a hydrogen atom; an optionally substituted alkyl; an optionally substituted alkenyl; an optionally substituted alkynyl; an optionally substituted cycloalkyl; an optionally substituted cycloalkenyl; an optionally substituted cycloalkynyl; an optionally substituted heterocyclyl group; or **R₅** and **R₆** can be linked together with the nitrogen atom to which they are covalently linked to form an optionally substituted heterocyclyl group ;
• **R₇** can be selected from an optionally substituted alkyl; a haloalkyl; a fluoroalkyl; an optionally substituted alkenyl; an optionally substituted alkynyl; an optionally substituted cycloalkyl; an optionally substituted cycloalkenyl; an optionally substituted cycloalkynyl; an optionally substituted heterocyclyl group;
• n is an integer which can have any one of the values 0, 1, 2, 3 or 4;
• m is an integer which can have any one of the values 0, 1, 2, 3, 4 or 5 for formula (II); and m is an integer which can have any one of the values 0, 1, 2, 3 or 4 for formula (III) and (IV);
and pharmaceutically acceptable salt, hydrates, solvate, prodrugs, polymorphs, tautomers, isotopic variants, isomers, stereoisomers or mixtures of stereoisomers thereof.

10. The combination according to claim 9, in which **R₁**, **R₂**, **R₃** and **R₄** are as defined in any of claims 2 to 8 for formula (II) or (III) and in which **R₁**,and **R₄** are as defined in any of claims 2, 3, 7 and 8 for formula (IV).

11. The combination according to claim 1, in which the compound of formula (I) is chosen from the following compounds:
2-(4-chlorophenylamino)-4-(4-*tert*-butylaminopiperidin-1-yl)-quinoline of formula (**Ia**) (**1-5**) 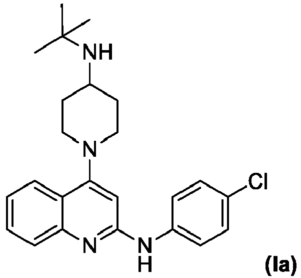
2-(4-chlorobenzylamino)-4-(4-*tert*-butylaminopiperidin-1-yl)-quinoline of formula (**Ib**) (**2-2**) 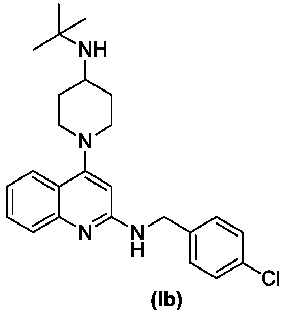
or a pharmaceutically acceptable salt, solvate or prodrug thereof.

12. The combination according to claim 1, in which the compound of formula (I) is chosen from 2-(4-chlorophenylamino)-4-(4-*tert*-butylaminopiperidin-1-yl)-quinoline hydrochloride salt (**1-6**) and 2-(4-chlorobenzylamino)-4-(4-*tert*-butylaminopiperidin-1-yl)-quinoline hydrochloride salt (**2-3**).

13. The combination according to any one of claims 1 to 12, in which the MEK inhibitor is chosen from the group consisting of Binimetinib [606143-89-9], Cobimetinib [934660-93-2], Selumetinib [606143-52-6], Trametinib [871700-17-3], AZD8330 [869357-68-6], BI-847325 [1207293-36-4], GDC-0623 [1168091-68-6], Mirdametinib (PD0325901) [391210-10-9], PD184352 (CI-1040) [212631-79-3], Pimasertib (AS-703026) [1236699-92-5], Refametinib [923032-37-5], TAK-733 [1035555-63-5], BIX 02188 [1094614-84-2], BIX 02189 [1265916-41 - 3], Honokiol [35354-74-6], Myricetin [529-44-2], PD98059 [167869-21-8], PD318088 [391210-00-7], SL-327 [305350-87-2], U0126 [109511-58-2] or a pharmaceutically acceptable salt or solvate thereof.

14. The combination according to any one of claims 1 to 13, in which the MEK inhibitor is chosen from the group consisting of Binimetinib [606143-89-9], Cobimetinib [934660-93-2], Selumetinib [606143-52-6], Trametinib [871700-17-3] or a pharmaceutically acceptable salt or solvate thereof.

15. The combination according to any one of claims 1 to 14, which comprises 2-(4-chlorobenzylamino)-4-(4-*tert*-butylaminopiperidin-1-yl)-quinoline of formula (**Ib**) (**2-2**), or a salt, solvate or prodrug thereof, and Trametinib [871700-17-3] or a pharmaceutically acceptable salt or a solvate thereof.

16. A pharmaceutical composition comprising a therapeutically effective amount of a combination according to any one of claims 1 to 15, comprising a compound of formula (I) and its pharmaceutically acceptable salts, hydrates, solvate, prodrugs, polymorphs, tautomers, isotopic variants, isomers, stereoisomers or mixtures of stereoisomers thereof and a MEK inhibitor or a pharmaceutically acceptable salt or solvate thereof, and a pharmaceutically acceptable carrier, where each component of the combination can be contained in a separate pharmaceutical composition.

17. A combination comprising a compound of formula (I) or a pharmaceutically acceptable salt or solvate thereof and a MEK inhibitor or a pharmaceutically acceptable salt or solvate thereof according to any one of claims 1 to 16, for use in the treatment and/or prevention and/or decrease of liver cancers related diseases.

18. A combination for use according to claim 17, comprising a compound of formula (II), (III) or (IV) or a pharmaceutically acceptable salt or solvate thereof as defined in claims 9 or 10.

19. A combination for use according to claim 17 comprising a compound of formula (I) chosen from 2-(4-chlorophenylamino)-4-(4-*tert*-butylaminopiperidin-1-yl)-quinoline hydrochloride salt (**1-6**) and 2-(4-chlorobenzylamino)-4-(4-*tert*-butylaminopiperidin-1-yl)-quinoline hydrochloride salt (**2-3**).

20. A combination for use according to claim 17 which is the combination of 2-(4-chlorobenzylamino)-4-(4-*tert*-butylaminopiperidin-1-yl)-quinoline of formula (**Ib**) (**2-2**), or a pharmaceutically acceptable salt, solvate or prodrug thereof, and Trametinib [871700-17-3] or a pharmaceutically acceptable salt or solvate thereof.

21. A combination for use according to any one of claims 17 to 20, in which each component of the combination can be for simultaneous, separate or sequential use in liver cancer therapy.

22. A combination for use according to claim 17 to 21, in which the liver cancers related disease is selected from hepatocarcinoma, hepatoblastoma and cholangiocarcinoma.

23. A combination for use according to any one of claims 17 to 22, in which the liver cancers related disease is selected from a intrahepatic cholangiocarcinoma and a extrahepatic cholangiocarcinoma.

24. A combination for use according to any one of claims 17 to 22, in which the liver cancers related disease is a hepatocarcinoma.

25. A kit comprising the combination of a compound of formula (I) or a pharmaceutically acceptable salt or solvate thereof and a MEK inhibitor or a pharmaceutically acceptable salt or solvate thereof, as defined in any one of claims 1 to 16, in which both or either of the components of the combination, are /is in the form of a pharmaceutical composition which may be administered simultaneously, separately or sequentially.
